(19) Europäisches Patentamt
European Patent Office
Office européen des brevets

(11) Publication number: 0 176 294 B1

# (12) EUROPEAN PATENT SPECIFICATION

(45) Date of publication of patent specification: 05.06.91

(51) Int. Cl.[5]: C07C 251/32, A01N 35/10

(21) Application number: 85306535.7

(22) Date of filing: 13.09.85

The file contains technical information submitted after the application was filed and not included in this specification

## (54) Cyclohexane-1,3-dione derivatives, compositions containing them, processes for preparing them, and their use as herbicides and plant growth regulators.

(30) Priority: 24.09.84 AU 7274/84

(43) Date of publication of application: 02.04.86 Bulletin 86/14

(45) Publication of the grant of the patent: 05.06.91 Bulletin 91/23

(84) Designated Contracting States: BE CH DE FR GB IT LI NL

(56) References cited:
EP-A- 0 086 588
EP-A- 0 124 992
AU-B- 503 917
DE-A- 3 239 071
US-A- 3 950 420

(73) Proprietor: ICI AUSTRALIA LIMITED
1 Nicholson Street
Melbourne Victoria 3001(AU)

(72) Inventor: Farquharson, Graeme John
8 Prince Edward Avenue
Carlton New South Wales 2218(AU)
Inventor: Watson, Keith Geoffrey
18 Hamilton Avenue
Blackburn North Victoria 3130(AU)
Inventor: Bird, Graham John
14 Roseberry Street
Ascot Vale Victoria 3032(AU)

(74) Representative: Downes, John Edward et al
Imperial Chemical Industries PLC Legal Department: Patents Po Box 6
Welwyn Garden City Herts, AL7 1HD(GB)

EP 0 176 294 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to organic compounds having biological activity and in particular to organic compounds having herbicidal properties and plant growth regulating properties, to processes for the preparation of such compounds, to intermediates useful in the preparation of such compounds and to herbicidal compositions and processes utilizing such compounds and to plant growth regulating compositions and processes utilizing such compounds.

The use of certain cyclohexane-1,3-dione derivatives as grass herbicides is known in the art. For example, the "Pesticide Manual" (C R Worthing Editor, The British Crop Protection Council, 6th Edition 1979) describes the cyclohexane-1,3-dione derivative known commercially as alloxydim-sodium (methyl 3-[1-(allyloxyimino)butyl]-4-hydroxy-6,6-dimethyl-2-oxocyclohex-3-ene carboxylate) and its use as a grass herbicide. This compound is disclosed in Australian Patent No 464 655 and its equivalents such as UK Patent No 1 461 170 and US Patent No 3 950 420.

More recently, at the 1980 British Crop Protection Conference ("1980 British Crop Protection Conference - Weeds, Proceedings Vol 1, Research Reports", pp 39 to 46, British Crop Protection Council, (1980), a new cyclohexane-1,3-dione grass herbicide code named NP 55 (2-N-ethoxybutrimidoyl)-5-(2-ethylthiopropyl)-3-hydroxy-2-cyclohexen-1-one) was announced. This compound is disclosed in Australian Patent No 503 917 and its equivalents.

From European Patent Application No 086 588, it is also known that 5-indanyl- and 5-tetralinyl-cyclohexane-1,3-dione derivatives exhibit useful herbicidal properties. It has now been found that a new group of 5-(oxoindanyl or oxotetralinyl) cyclohexane -1,3-dione derivatives exhibit particularly strong general grass-killing properties.

Accordingly the invention provides a compound of formula I or an isomer thereof:

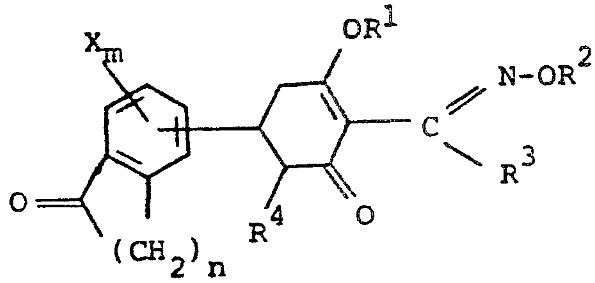

I

wherein:
n is an integer selected from 2 to 4;
m is zero or an integer selected from 1 to 3;
X, which may be the same or different, are independently selected from the group consisting of: halogen; $C_1$ to $C_6$ alkyl; $C_1$ to $C_6$ alkoxy; and $C_1$ to $C_6$ alkylthio;
$R^1$ is selected from the group consisting of:
hydrogen; an acyl group; and an inorganic or organic cation;
$R^2$ is selected from the group consisting of: $C_1$ to $C_6$ alkyl; $C_2$ to $C_6$ alkenyl; $C_2$ to $C_6$ haloalkenyl; $C_2$ to $C_6$ alkynyl; $C_3$ to $C_6$ haloalkynyl; substituted $C_1$ to $C_6$ alkyl wherein the alkyl group is substituted with a substituent selected from the group consisting of halogen, $C_1$ to $C_6$ alkoxy, $C_1$ to $C_6$ alkylthio, phenyl and substituted phenyl wherein the benzene ring is substituted with from one to three substituents selected from the group consisting of halogen, $C_1$ to $C_6$ alkyl, $C_1$ to $C_6$ haloalkyl, $C_1$ to $C_6$ alkoxy, $C_1$ to $C_6$ alkylthio;
$R^3$ is selected from the group consisting of: $C_1$ to $C_6$ alkyl; $C_1$ to $C_6$ fluoroalkyl; $C_2$ to $C_6$ alkenyl; $C_2$ to $C_6$ alkynyl; and phenyl; and
$R^4$ is selected from the group consisting of: hydrogen; halogen; $C_1$ to $C_6$ alkyl; cyano; and ($C_1$ to $C_6$ alkoxy) carbonyl.

When in the compound of formula I $R^1$ is chosen from acyl the nature of the acyl group is not narrowly critical. Although not intending to be bound by theory, it is believed that when $R^1$ is acyl the acyl group may be removed in the plant by hydrolysis to give the corresponding compound of formula I in which $R^1$ is hydrogen. Suitable acyl groups include: alkanoyl, for example $C_2$ to $C_6$ alkanoyl; aroyl, for example benzoyl and substituted benzoyl wherein the benzene ring is substituted with from one to three substituents chosen from the group consisting of halogen, nitro, cyano, $C_1$ to $C_6$ alkyl, $C_1$ to $C_6$ haloalkyl, $C_1$ to $C_6$ alkoxy and

$C_1$ to $C_6$ alkythio.

When in the compound of formula I $R^1$ is chosen from an inorganic or organic cation the nature of the cation is not narrowly critical. Although not intending to be bound by theory, it is believed that when $R^1$ is a cation the cation may be removed in the plant to give a compound of formula I wherein $R^1$ is hydrogen. Suitable inorganic cations include the alkali and alkaline earth metal ions, heavy metal ions including the transition metal ions, and the ammonium ion. Suitable organic cations include the cation $R^9R^{10}R^{11}R^{12}N^+$ wherein $R^9$, $R^{10}$, $R^{11}$ and $R^{12}$ are independently chosen from the group consisting of: hydrogen; $C_1$ to $C_{10}$ alkyl; substituted $C_1$ to $C_{10}$ alkyl wherein the alkyl group is substituted with a substituent chosen from the group consisting of hydroxy, halogen and $C_1$ to $C_6$ alkoxy; phenyl; benzyl; and the groups substituted phenyl and substituted benzyl wherein the benzene ring is substituted with from one to three substituents chosen from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio.

It should be recognized that when $R^1$ is hydrogen the compounds of the invention may exist in any one of four tautomeric forms as shown below wherein Ø represents the group

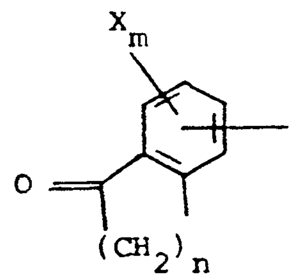

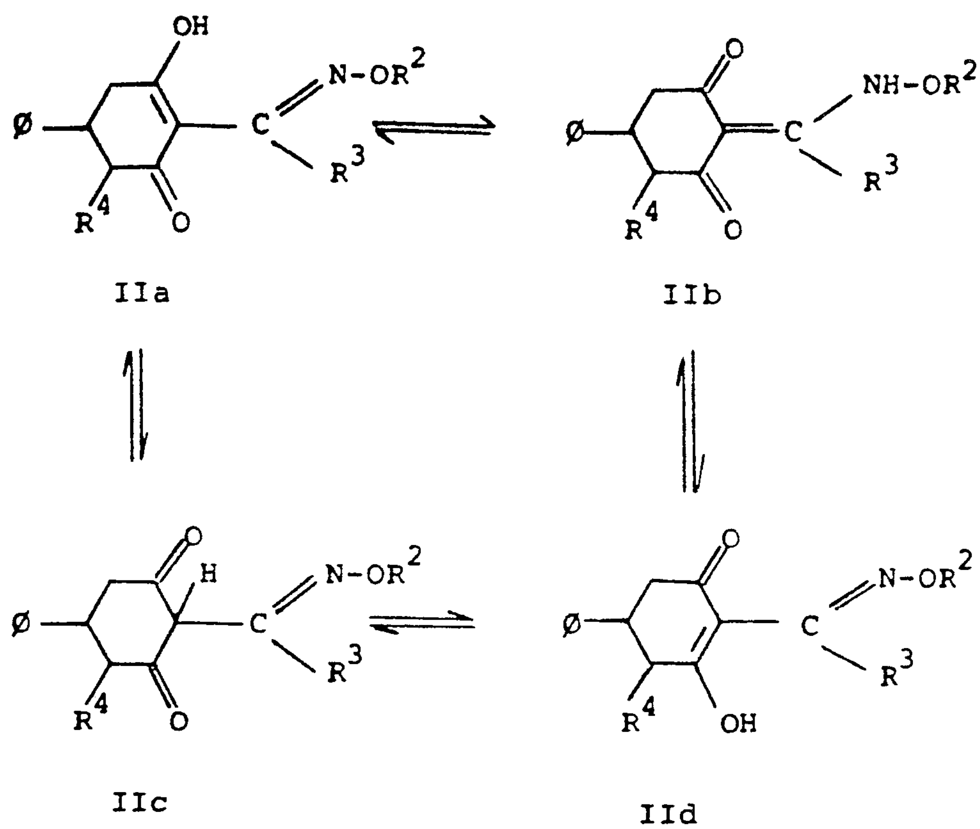

IIa

IIb

IIc

IId

The compounds of the invention include:
indanone derivatives of formula Ia; and

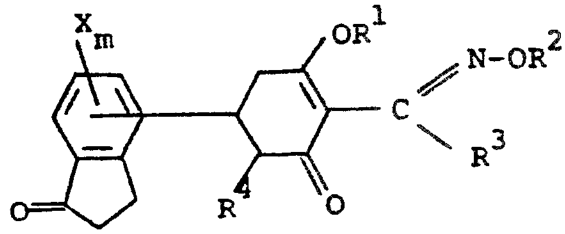

Ia

tetralone derivatives of formula Ib.

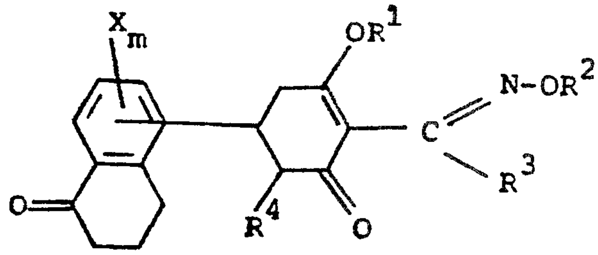

Ib

Preferred compounds of the invention include those compounds of formula I wherein:
n is an integer selected from 2 and 3;
m is an integer selected from 1 to 3;
X, which may be the same or different, are independently selected from the group consisting of: halogen; $C_1$ to $C_6$ alkyl; $C_1$ to $C_6$ alkoxy; and $C_1$ to $C_6$ alkylthio;
$R^1$ is selected from the group consisting of: hydrogen; $C_2$ to $C_6$ alkanoyl; benzoyl and substituted benzoyl wherein the benzene ring is substituted with from one to three substituents selected from the group consisting of halogen, nitro, $C_1$ to $C_6$ alkyl and $C_1$ to $C_6$ alkoxy; benzenesulfonyl and substituted benzenesulfonyl wherein the benzene ring is substituted with from one to three substituents selected from the group consisting of halogen, nitro, $C_1$ to $C_6$ alkyl and $C_1$ to $C_6$ alkoxy; and an inorganic or an organic cation selected from the alkali metals such as lithium, potassium and sodium, the alkaline earth metals such as magnesium, calcium and barium, the transition metals such as manganese, copper, zinc, iron, nickel, cobalt and silver, the ammonium ion and the tri- and tetra- (alkyl)ammonium ions wherein alkyl is selected from $C_1$ to $C_6$ alkyl and $C_1$ to $C_6$ hydroxyalkyl;
$R^2$ is selected from the group consisting of $C_1$ to $C_6$ alkyl, $C_2$ to $C_6$ alkenyl, $C_2$ to $C_6$ alkynyl, $C_1$ to $C_6$ haloalkyl, $C_2$ to $C_6$ halo alkenyl and $C_3$ to $C_6$ haloalkynyl;
$R^3$ is selected from $C_1$ to $C_6$ alkyl; and
$R^4$ is hydrogen.

More preferred compounds of the invention include those compounds of formula I wherein:
n is an integer selected from 2 and 3;
m is an integer selected from 1 to 3;
X, which may be the same or different, are independently selected from the group consisting of : chlorine; methyl; methoxy; and methylmercapto;
$R^1$ is selected from the group consisting of hydrogen, benzoyl and the alkali metals;
$R^2$ is selected from the group consisting of $C_1$ to $C_6$ alkyl, $C_1$ to $C_6$ haloalkyl, $C_2$ to $C_6$ alkenyl and $C_2$ to $C_6$ alkynyl;
$R^3$ is selected from the group consisting of $C_1$ to $C_6$ alkyl; and
$R^4$ is hydrogen.

Even more preferred compounds of the invention include those compounds of formula I wherein:
n is an integer selected from 2 and 3;
m is an integer selected from 1 to 3;
X, which may be the same or different, are independently selected from the group consisting of: chlorine; methyl; methoxy; and methylmercapto;
$R^1$ is selected from the group consisting of hydrogen and alkali metals;
$R^2$ is selected from the group consisting of $C_1$ to $C_3$ alkyl; $C_1$ to $C_3$ fluoroalkyl; allyl; and propargyl;
$R^3$ is selected from the group consisting of $C_1$ to $C_3$ alkyl; and
$R^4$ is hydrogen.

Examples of compounds embraced by the invention include:

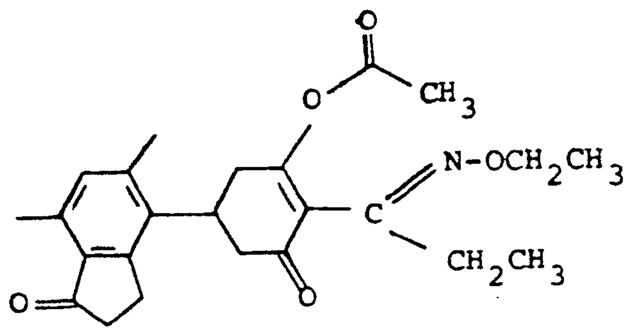

1

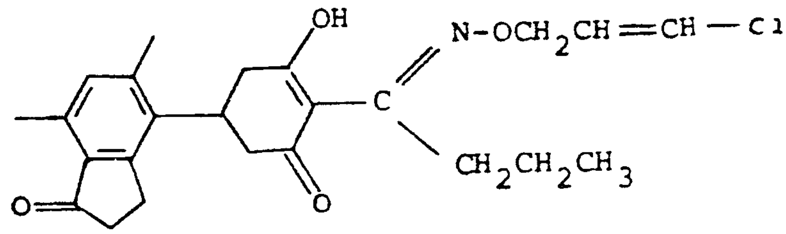

2

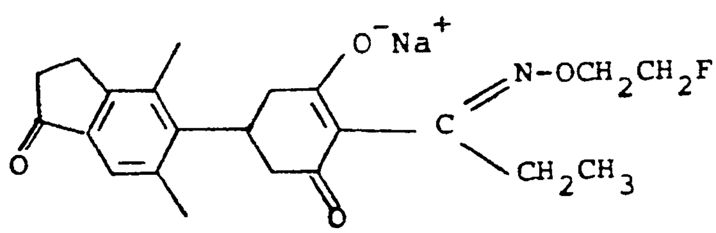

3

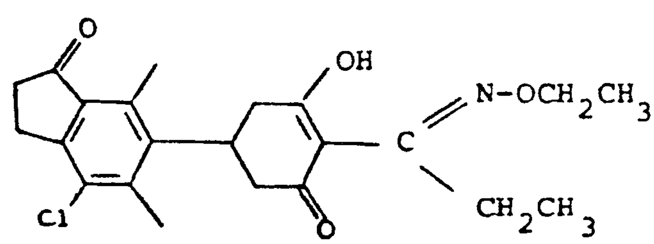

4

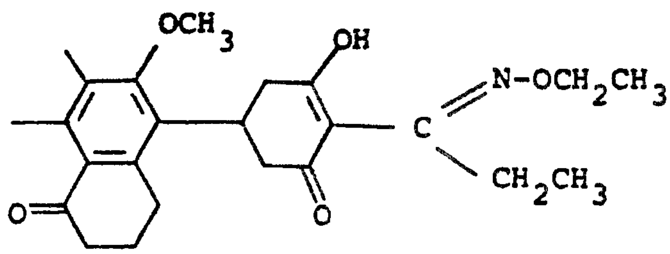

5

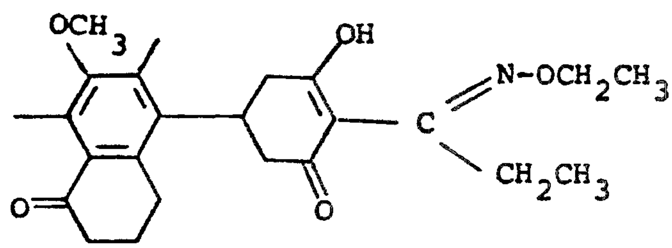

6

Specific examples of the compounds of the invention include the compounds listed in Table 1 below:

TABLE 1

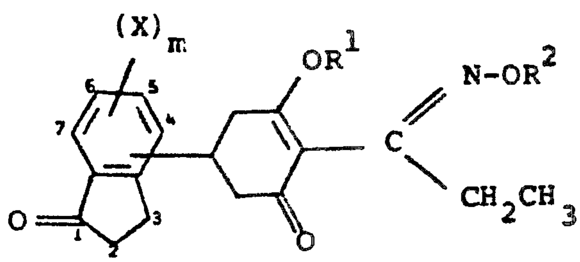

| Compound No | (X)m | Position of ring linkage | $R^1$ | $R^2$ |
|---|---|---|---|---|
| 7 | 5,7-$(CH_3)_2$ | 4 | H | $CH_2CH_3$ |
| 8 | 5,7-$(CH_3)_2$ | 6 | H | $CH_2CH_3$ |
| 9 | 5,7-$(CH_3)_2$ | 4 | H | $CH_2CH=CH_2$ |
| 10 | 5,7-$(CH_3)_2$ | 6 | H | $CH_2CH=CH_2$ |
| 11 | 5,7-$(CH_3)_2$ | 4 | Na | $CH_2CH_3$ |

The compounds of the invention may be prepared by a variety of methods and in a further aspect the invention provides methods for the preparation of compounds of formula I.

Conveniently the preparation of the compounds of the invention can be considered in four or five parts.

Part A involves the formation of a 5-arylcyclohexan-1,3-dione of formula IX. This reaction may be carried out in a two step process by:

(i) reacting, preferably in the presence of a base, an aldehyde derivative of formula V with acetone (IVa) or an acetone derivative of formula IVb to form a ketone derivative of formula VIa or VIb respectively; and reacting, preferably in the presence of a base, a ketone derivative of formula VIa with a malonic acid ester derivative of formula VIIa or a ketone derivative of formula VIb with a malonic acid ester of formula VIIb, to give an intermediate of formula VIIIa or VIIIb respectively which may be isolated or hydrolysed directly, preferably in the presence of an acid, to give a 5-arylcyclohexan-1,3-dione of formula IX, or reacting, preferably in the presence of a base, a ketone derivative of formula VIa with an alkanoic acid ester of formula VIIc to give a 5-arylcyclohexan-1,3-dione of formula IX;

(ii) reacting, preferably in the presence of a base, an aldehyde derivative of formula V with a malonic acid ester of formula VIIb to give an arylmethylidenemalonate derivative of formula VIc which is in turn reacted, preferably in the presence of a base, with an acetoacetic acid derivative of formula VIId to give an intermediate of formula VIIIc which may be isolated or hydrolysed directly, preferably in the presence of an acid, to give a 5-arylcyclohexan-1,3-dione of formula IX;

(iii) reacting, preferably in the presence of a base, an aldehyde derivative of formula V with an acetic acid ester of formula IVc to give a 2-arylalkenoate derivative of formula VId which is in turn reacted, preferably in the presence of a base, with an acetoacetic acid ester derivative of formula VIId to give an intermediate of formula VIIIa which may be isolated or hydrolysed directly, preferably in the presence of an acid, to give a 5-arylcyclohexan-1,3-dione of formula IX, or

(iv) reacting an aldehyde derivative of formula V with an ylide of formula IVd or IVe, wherein Ar is an aryl group, to form a derivative of formula VIa or VId respectively; and further reaction of the derivatives of formula VIa or VId as described above in parts (i) and (iii) respectively to give a 5-arylcyclohexan-1,3-dione of formula IX.

Part B involves reaction of a 5-arylcyclohexan-1,3-dione of formula IX with an acidic dehydrating agent to give a derivative of formula X. Suitable dehydrating agents include polyphosphoric acid and methanesulfonic acid.

Part C involves the acylation of a compound of formula X to give a 2-acyl-5-arylcyclohexan-1,3-dione derivative of formula XIV. The acylation reaction may be carried out by reacting a derivative of formula X with:

(v) an acid anhydride of formula XI in the presence of either an alkali metal salt of the corresponding acid of formula XII or an alkoxide salt of formula XIII, wherein M is an alkali metal ion and R is $C_1$ to $C_6$ alkyl;

(vi) an acid anhydride of formula XI in the presence of the corresponding acid of formula XV, preferably in the presence of a Lewis acid or Bronsted acid catalyst;

(vii) an alkali or alkaline earth metal hydride followed by reaction with an acid anhydride of formula XI or

an acid halide of formula XVI;

(viii) an acid anhydride of formula XI in the presence of a strong organic base such as 4-dimethylaminopyridine or imidazole.

Alternatively, this reaction may be carried out by:

(ix) reacting a derivative of formula X with an acid halide of formula XVI in the presence of a base to give an intermediate O-acyl derivative of formula XVII; and

(x) reacting the intermediate of formula XVII with a Lewis acid or Bronsted acid catalyst;

(xi) reacting the intermediate of formula XVII with a suitable strong organic base such as 4-dimethylaminopyridine or imidazole.

Part D involves the formation of a compound of the invention of formula I wherein $R^1$ is hydrogen, that is a compound of formula II. This reaction may be carried out either by reacting a 2-acyl-5-arylcyclohexane-1,3-dione derivative of formula XIV with:

(xii) an alkoxyamine derivative of formua XVIII, or

(xiii) hydroxylamine to give an intermediate oxime derivative of formula XIX and reacting that intermediate oxime derivative of formula XIX with an alkylating agent of formula XX, wherein L is a leaving group such as, for example, chloride, bromide, iodide, sulfate, nitrate, methyl sulfate, ethyl sulfate, tetrafluoroborate, hexafluorophosphate, hexafluoroantimonate, methanesulfonate, fluorosulfonate, fluoromethanesulfonate and trifluoromethanesulfonate.

Part E involves the formation of a compound of the invention of formula I where $R^1$ is a substituent other than hydrogen.

Compounds of the invention of formula I, wherein $R^1$ forms an acyl or sulfonyl derivative of a compound of formula II, may be prepared from the corresponding compounds of the invention of formula II by reacting with an acylation or sulfonylation reagent of formula XXI.

Compounds of the invention of formula I wherein $R^1$ is an inorganic or organic cation may be prepared from the compounds of the invention of formula I wherein $R^1$ is hydrogen, that is, compounds of formula II, by reacting said compounds of formula II with an inorganic or organic salt. For example, the compounds of formula I wherein $R^1$ is an alkali metal ion may be prepared by reacting the appropriate compound of formula II with the appropriate alkali metal hydroxide or alkoxylate. The compounds of formula I wherein $R^1$ is a transition metal ion or an organic cation may similarly be prepared by reacting the appropriate compound of formula II with an appropriate transition metal salt or organic base. Alternatively, the compounds of formula I wherein $R^1$ is a transition metal ion or an organic cation may be prepared by reacting the appropriate compound of formula I wherein $R^1$ is an alkali metal ion with an appropriate transition metal salt or organic salt.

Accordingly, in a further aspect the invention provides a process for the preparation of a compound of formula I, as hereinbefore defined, which process comprises:

reacting 2-acyl-5-arylcyclohexane-1,3-dione derivative of formula XIV with an alkoxyamine derivative of formula XVIII to give a compound of the invention of formula II or reacting the 2-acyl-5-arylcyclohexane-1,3-dione derivative of formula XIV with hydroxylamine and alkylating the oxime intermediate of formula XIX with an alkylating agent of formula XX, wherein L is a leaving group, to give a compound of the invention of formula II; and optionally

reacting the compound of the invention of formula II with a compound of formula XXI wherein L is a leaving group, to give a compound of the invention of formula I.

Certain of the intermediate compounds of formulae VI, VIII, IX, X, XIV, XVII and XIX are novel compounds and therefore in further embodiments the invention provides novel compounds of formulae VI, VIII, IX, X, XIV, XVII and XIX and processes for the preparation thereof.

It is not possible to use standard methods such as the Vilsmeier formylation or dichloromethyl methyl ether to prepare formylindanones or formyltetralones which would be the expected starting materials for the preparation of the cyclohexan-1,3-diones of formula X. It was necessary to find a means of forming the indanone or tetralone ring system after the cyclohexane-1,3-dione ring formation.

Accordingly, in a further aspect the invention provides a process for the preparation of 5-(1-oxoindanyl)- and 5-(1-oxotetrahydronaphthyl) cyclohexan -1,3-dione derivatives of formula X, which process comprises reacting a 5-(carboxyalkyl phenyl) cyclohexan-1,3-dione of formula IX with an acidic dehydrating agent.

The structures of the compounds described above are detailed on the following pages wherein ∅ represents the group.

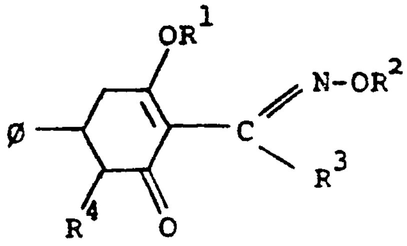

I

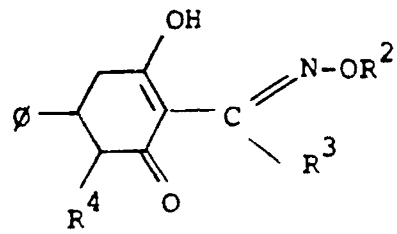

II

$CH_3COCH_3$

IVa

$CH_3COCH_2R^4$

IVb

$CH_3CO_2R$

IVc

$Ar_3P{=}CHCOCH_3$

IVd

$Ar_3P{=}CHCO_2R$

IVe

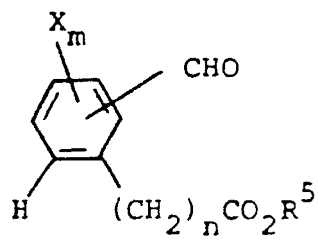

V

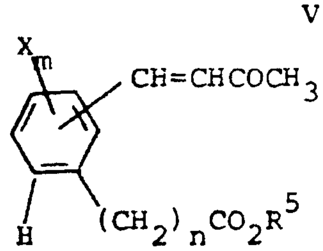

VIa

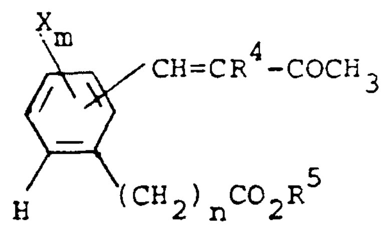

VIb

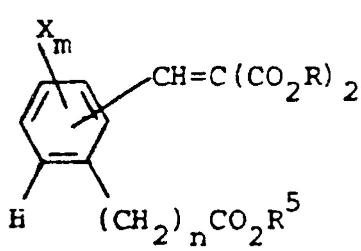

VIc

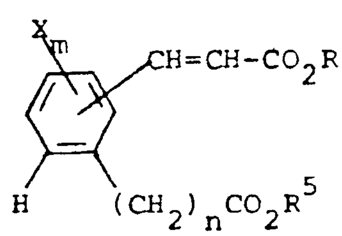

VId

$R^4CH(CO_2R)_2$

VIIa

$CH_2(CO_2R)_2$

VIIb

$R^4CH_2CO_2R$

VIIc

$CH_3COCHR^4CO_2R$

VIId

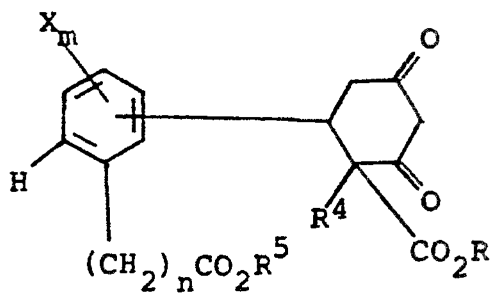

VIIIa

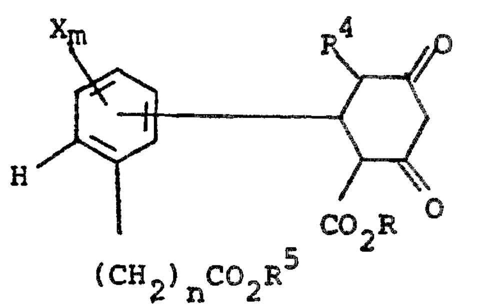

VIIIb

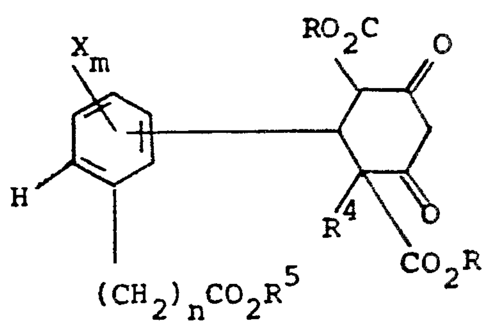

VIIIc

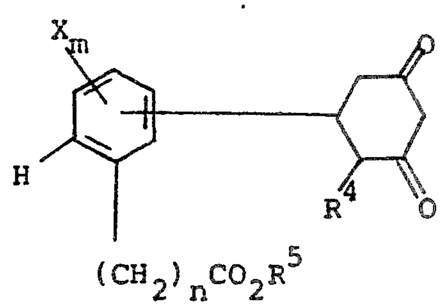

IX

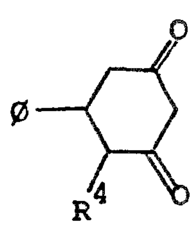

X

$(R^3CO)_2O$ XI

$R^3CO_2M$ XII

ROM XIII

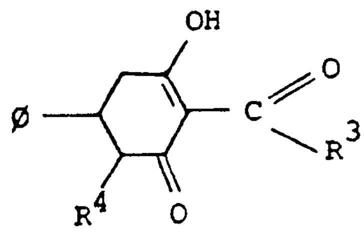

XIV

$R^3CO_2H$ XV

$R^3COhal$ XVI

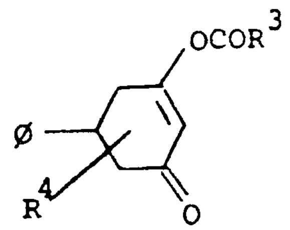

XVII

$H_2NOR^2$ XVIII

$R^2L$ XX

$R^1L$ XXI

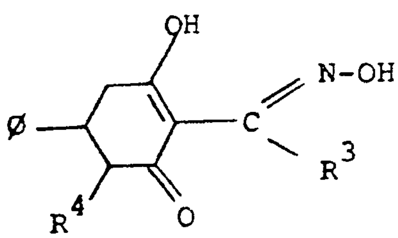

XIX

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of formula I are selectively active against monocotyledonous plants, dicotyledonous plants being relatively unaffected by rates of application of the compounds of the invention which are severely damaging or lethal to other plant species.

Moreover, certain of the compounds of formula I are selectively active within the group of monocotyledonous plants and may be used at a rate sufficient to control monocotyledonous weeds in cultivated crops,

especially wild grasses in cereal crops. Certain of such compounds of the invention are especially useful in the control of wild grasses such as wild oats and rye grass in crops of cultivated monocotyledonous plants such as wheat, barley and other varieties of cereals.

Accordingly, in yet a further aspect the invention provides a process for controlling monocotyledonous weeds in cultivated crops, especially wild grasses in cereal crops such as wheat, which process comprises applying to the crop, or to the growth medium of the crop, a compound of formula I, as hereinbefore defined, in an amount sufficient to severely damage or kill the weeds but insufficient to damage the crop substantially.

The compounds of formula I may be applied directly to the plant (post-emergence application) or to the soil before the emergence of the plant (pre-emergence application). However, the compounds are, in general, more effective when applied to the plant post-emergence.

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides growth inhibiting, plant damaging, or plant killing compositions comprising a compound of formula I as hereinbefore defined and an inert carrier therefor.

Certain of the compounds of formula I exhibit useful plant growth regulating activity. For example, while compounds of formula I are selectively active herbicides against wild grasses in crops of cultivated plants at some rates of application they exhibit plant growth regulating effects in said crops.

Plant growth regulating effects may be manifested in a number of ways. For example, suppression of apical dominance, stimulation of auxiliary bud growth, stimulation of early flowering and seed formation, enhancement of flowering and increase in seed yield, stem thickening, stem shortening and tillering. Plant growth regulating effects shown in compounds of the invention may include, for example, tillering and stem shortening in crops such as wheat and barley.

Accordingly in a still further aspect the invention provides a process for regulating the growth of a plant which process comprises applying to the plant, to the seed of the plant, or to the growth medium of the plant, an effective amount of a compound of formula I, as hereinbefore defined.

To effect the plant growth regulating process of the present invention the compounds of formula I may be applied directly to the plant (post-emergence application)or to the seed or soil before the emergence of the plant (pre-emergence) application.

The compounds of formula I may be used on their own to regulate the growth of plants but in general are preferably used in the form of a composition comprising a compound of the invention in admixture with a carrier comprising a solid or liquid diluent. Therefore, in a still further aspect the invention provides plant growth regulating compositions comprising a compound of formula I as hereinbefore defined and an inert carrier therefor.

The compositions of the present invention may be in the form of solids, liquids or pastes. The compositions include both dilute compositions which are ready for immediate use and concentrated compositions which may require dilution before use. Therefore, the concentration of the active ingredient in the compositions of the present invention will vary depending on the types of formulation and whether the composition is ready for use such as, for example, a dust formulation or an aqueous emulsion or whether the composition is a concentrate such as, for example, an emulsifiable concentrate or a wettable powder, which is suitable for dilution before use. In general the compositions of the present invention comprise from 1 ppm to 99% by weight of active ingredient.

The solid compositions may be in the form of powders, dusts, pellets, grains, and granules wherein the active ingredient is mixed with a solid diluent. Powders and dusts may be prepared by mixing or grinding the active ingredient with a solid carrier to give a finely divided composition. Granules, grains and pellets may be prepared by bonding the active ingredient to a solid carrier, for example, by coating or impregnating the preformed granular solid carrier with the active ingredient or by agglomeration techniques.

Examples of solid carriers include: mineral earths and clays such as, for example, kaolin, bentonite, kieselguhr, Fuller's earth, Attaclay, diatomaceous earth, bole, loess, talc, chalk, dolomite, limestone, lime, calcium carbonate, gypsum, calcium sulfate, pyrophyllite, silicic acid, silicates and silica gels; fertilizers such as, for example, ammonium sulfate, ammonium phosphate, ammonium nitrate and urea; natural products of vegetable origin such as, for example, grain meals and flours, bark meals, wood meals, nutshell meals and cellulosic powders; and synthetic polymeric materials such as, for example, ground or powdered plastics and resins.

Alternatively, the solid compositions may be in the form of dispersible or wettable dusts, powders, granules or grains wherein the active ingredient and the solid carrier are combined with one or more surface active agents which act as wetting, emulsifying and/or dispersing agents to facilitate the dispersion of the

active ingredient in liquid.

Examples of surface active agents include those of the cationic, anionic and non-ionic type. Cationic surface active agents include quaternary ammonium compounds, for example, the long chain alkylammonium salts such as cetyltrimethylammonium bromide. Anionic surface active agents include: soaps or the alkali metal, alkaline earth metal and ammonium salts of fatty acids; the alkali metal, alkaline earth metal and ammonium salts of ligninsulfonic acid; the alkali metal, alkaline earth metal and ammonium salts of arylsulfonic acids including the salts of naphthalenesulfonic acids such as butylnaphthalenesulfonic acids, the di- and tri- isopropylnaphthalenesulfonic acids, the salts of the condensation products of sulfonated naphthalene and naphthalene derivatives with formaldehyde, the salts of the condensationproducts of sulfonated naphthalene and naphthalene derivatives with phenol and formaldehyde, and the salts of alkylarylbenzenesulfonic acids such as dodecylbenzenesulfonic acid; the alkali metal, alkaline earth metal and ammonium salts of the long chain mono esters of sulfuric acid or alkylsulfates such as laurylsulfate and the mono esters of sulfuric acid with fatty alcohol glycol ethers. Nonionic surface active agents include: the condensation products of ethylene oxide with phenols and alkylphenols such as isooctylphenol, octylphenol and nonylphenol; the condensation products of ethylene oxide with castor oil; the partial esters derived from long chain fatty acids and hexitol anhydrides, for example sorbitan monolaurate, and their condensation products with ethylene oxide; ethylene oxide/propylene oxide block copolymers; lauryl alcohol polyglycol ether acetal; and the lecithins.

The liquid compositions may comprise a solution or dispersions of the active ingredient in a liquid carrier optionally containing one or more surface active agents which act as wetting, emulsifying and or dispersing agents. Examples of liquid carriers include: water; mineral oil fractions such as, for example, kerosene, solvent naphtha, petroleum, coal tar oils and aromatic petroleum fractions; aliphatic, cycloaliphatic and aromatic hydrocarbons such as, for example, paraffin, cyclohexane, toluene, the xylenes, tetrahydronaphthalene and alkylated naphthalenes; alcohols such as, for example, methanol, ethanol, propanol, isopropanol, butanol, cyclohexanol and propylene glycol; ketones such as, for example, cyclohexanone and isophorone; and strongly polar organic solvents such as, for example, dimethylformamide, dimethylsulfoxide, N-methylpyrrolidone and sulfolane.

A preferred liquid composition comprises an aqueous suspension, dispersion or emulsion of the active ingredient which is suitable for application by spraying, atomizing or watering. Such aqueous compostions are generally prepared by mixing concentrated compositions with water. Suitable concentrated compositions include emulsion concentrates, pastes, oil dispersions, aqueous suspensions and wettable powders. The concentrates are usually required to withstand storage for prolonged periods and after such storage to be capable of dilution with water to form aqueous preparations which remain homogeneous for a sufficient time to enable them to be applied by conventional spray equipment. The concentrates conveniently contain from 10 to 99%, preferably 10 to 60%, by weight of active ingredient.

Emulsion or emulsifiable concentrates are conveniently prepared by dissolving the active ingredient in an organic solvent containing one or more surface active agents and optionally an oil. Oil dispersions may be prepared by grinding together the active ingredient, a hydrocarbon oil, and one or more surface active agents. Aqueous suspension concentrates may conveniently be prepared by ball milling a mixture of the active agent and preferably at least one suspending agent. Suitable suspending agents include:
hydrophilic colloids such as, for example, poly(N-vinylpyrrolidone), sodium carboxymethylcellulose and the vegetable gums, gum acacia and gum tragacanth; hydrated colloidal mineral silicates such as, for example, montmorillonite, beidellite, nontronite, hectorite, saponite, sauconite and bentonite; other cellulose derivatives; and poly(vinyl alcohol). Wettable powder concentrates may conveniently be prepared by blending together the active ingredient, one or more surface active agents, one or more solid carriers and optionally one or more suspending agents and grinding the mixture to give a powder having the required particle size.

The aqueous suspensions, dispersions or emulsions may be prepared from the concentrated compositions by mixing the concentrated compositions with water optionally containing surface active agents and or oils.

It should be noted that the compounds of the invention of formula I wherein $R^1$ is hydrogen are acidic. Therefore, the compounds of formula I may be formulated and applied as the salts of organic or inorganic bases. In formulating and employing the compounds of formula I in the form of their salts either the salts per se, that is the compounds of formula I wherein $R^1$ is an inorganic or an organic cation, may be used in the formulation or the compounds of formula I wherein $R^1$ is hydrogen may be used in the formulation and the salts generated in situ by the use of the appropriate organic or inorganic base.

The mode of application of the compositions of the invention will depend to a large extent on the type of composition used and the facilities available for its application. Solid compositions may be applied by dusting or any other suitable means for broadcasting or spreading the solid. Liquid compositions may be

applied by spraying, atomizing, watering, introduction into the irrigation water, or any other suitable means for broadcasting or spreading the liquid.

The rate of application of the compounds of the invention will depend on a number of factors including, for example, the compound chosen for use, the identity of the plants whose growth is to be inhibited the formulations selected for use and whether the compound is to be applied for foliage or root uptake. As a general guide, however, an application rate of from 0.005 to 20 kilograms per hectate is suitable while from 0.01 to 5.0 kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may not be sufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad-leaved weeds. A second preferred class is of mixtures comprising a contact herbicide.

Example of useful complementary herbicides include:

A. benzo-2,1,3,-thiadiazin-4-one-2,2-dioxides such as 3-isopropylbenzo-2,1,3-thiadiazin-4-one-2,2-dioxide (common name bentazon);

B. hormone herbicides and in particular the phenoxyalkanoic acids such as 4-chloro-2-methylphenoxy acetic acid (common name MCPA), 2-(2,4-dichlorophenoxy)propionic acid (common name dichlorprop), 2,4-dichlorophenoxy acetic acid (common name 2,4,-D) 2,4,5-trichlorophenoxyacetic acid (common name 2,4,5-T), 4-(4-chloro-2-methylphenoxy)butyric acid (common name MCPB), 4-(2,4-dichlorophenoxy)-butyric acid (common name 2,4-DB), 2-(4-chloro-2-methylphenoxy)propionic acid (common name mecoprop), and their derivatives (eg salts, esters, amides and the like);

C. 3-[4-(4-halophenoxy)phenyl]-1,1-dialkylureas such as 3-[4-(4-chlorophenoxy)phenyl]-1,1-dimethylurea (common name chloroxuron);

D. dinitrophenols and their derivatives (eg acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-m-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenylurea herbicides such as N'-(3,4-dichlorophenyl)-N,N-dimethylurea (common name diuron) and N,N-dimethyl-N'-[3-(trifluoromethyl)phenyl]urea (common name fluometuron);

G. phenylcarbamoyloxyphenylcarbamates such as 3-[(methoxycarbonyl)amino]phenyl (3-methylphenyl)-carbamate (common name phenmedipham) and 3-[(ethoxycarbonylamino]phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-sec-butyl-6-methyluracil (common name bromacil) and 3-tert-butyl-5-chloro-6-methyluracil (common name terbacil);

J. triazine herbicides such as 2-chloro-4-ethylamino-6-(iso-propylamino)-1,3,5-triazine (common name atrazine). 2-chloro-4,6-di(ethylamino)-1,3,5-triazine (common name simazine) and 2-azido-4-(iso-propylamino)-6-methylthio-1,3,5-triazine (common name aziproptryne);

K. 1-alkoxy-2-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. pyridine herbicides such as 3,6-dichloropicolinic acid (common name clopyralid) and 4-amino-3,5,6-trichloropicolinic acid (common name picloram);

M. 1,2,4-triazin-5-one herbicides such as 4-amino-4,5-dihydro-3-methyl-6-phenyl-1,2,4-triazine-5-one (common name metamitron) and 4-amino-6-tert-butyl 4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoicacid (common name dicamba) and 3-amino-2,5-dichlorobenzoic acid (common name

chloramben);

O. anilide herbicides such as N-butoxymethyl- α - chloro-2',6'-diethylacetanilide (common name butachlor), the corresponding N-methoxy compound (common name alachlor), the corresponding N-isopropyl compound (common name propachlor) and 3',4'-dichloropropionanilide (common name propanil);

P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil);

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether;

S. N-(heteroarylaminooarbonyl)benzenesulfonamides such as 2-chloro-N-[(4-methoxy-6-methyl-1,3,5-triazin-2-yl)aminocarbonyl]benzenesulfonamide (commonly known as DPX 4189);

T. Aryloxyphenoxypropionate herbicides such as butyl 2-[4(5-trifluoromethyl-2-pyridyloxy)phenoxy] propionate (common name fluazifop) and methyl 2-[4(2,4-dichlorophenoxy)phenoxy]propionate (common name diclofop); and

U. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

V. bipyridylium herbicides such as those in which the active entity is the 1,1'-dimethyl-4,4'-dipyridylium ion (common name paraquat) and those in which the active entity is the 1,1'-ethylene-2,2'-dipyridylium ion (common name diquat);

W. organoarsenical herbicides such as monosodium methanearsonate (common name MSMA); and

X. amino acid herbicides such as N-(phosphonomethyl)glycine (common name glyphosate) and its salts and esters.

The invention is now illustrated by but in no way limited to the following example.

Example 1

5-(6,8-Dimethyl-7-methoxy-1-oxo-1,2,3,4-tetrahydronaphth-5-yl)-2-[1-(ethoxyimino)propyl]-3-hydroxycyclohex-2-en-1-one (6)

i) Succinic anhydride (22.06 gm) was added to a mixture of 2,6-dimethylanisole (30 g) and anhydrous aluminium trichloride (59 g) in 1,2-dichloroethane (200 ml) at 0° C. The mixture was stirred for 0.5 hr at 0° C and at room temperature for 4 hr. The mixture was poured onto ice-concentrated hydrochloric acid. After stirring vigorously for 5 min, the mixture was extracted with ethyl acetate. The dried ($MgSO_4$) organic fraction was evaporated to give 4-(3,5-dimethyl-4-methoxyphenyl)-4-oxobutyric acid as white crystals, mp 108° C.

ii) A mixture of 4-(3,5-dimethyl-4-methoxyphenyl)-4-oxobutyric acid (13 g), zinc amalgam [prepared from zinc (15 g) and mercuric chloride (1.5 g)], glacial acetic acid (70 ml), water (70 ml) and concentrated hydrochloric acid (170 ml) was heated at reflux for 12 hr. The cooled mixture was filtered and the filtrate was poured into water (1 litre). The mixture was extracted with ethyl acetate and the organic fraction was washed four times with water. The dried ($MgSO_4$) organic fraction was evaporated to give 4-(3,5-dimethyl-4-hydroxyphenyl)butyric acid as a pale yellow oil. Pmr spectrum ($CDCl_3$; δ in ppm): 1.79-2.64 (6H,m); 2.22 (6H,s); 6.79 (2H,s); 7.6 (2H,brs).

iii) Dimethyl sulfate (6 ml) was added to a well stirred mixture of 4-(3,5-dimethyl-4-hydroxyphenyl)butyric acid (9.4 g) and sodium hydroxide (2.69 g) in water (150 ml) at 5-10° C. The mixture was stirred for 20 mins at 10° C and was then heated to reflux for 1 hr. The cooled mixture was poured into water (200 ml) which was then extracted with diethyl ether. The dried ($MgSO_4$) organic fraction was evaporated to give methyl 4-(3,5-dimethyl-4-methoxyphenyl)butyrate as a pale yellow oil. Pmr spectrum ($CDCl_3$; δ in ppm): 1.89-2.61 (6H,m); 2.25 (6H,s); 3.66 (3H,s); 3.69 (3H,s); 6.81 (2H,s).

iv) Dichloromethyl methyl ether (5.3 g equiv) was added slowly to a well stirred mixture of methyl 4-(3,5-dimethyl-4-methoxyphenyl)butyrate (9.75 g) and titanium tetrachloride (31 g equiv) in dichloromethane (200 ml) at 0-5° C. The mixture was stirred for 1 hr at 5° C and at room temperature for 2 hrs. The mixture was poured onto ice-water which was then extracted with dichloromethane. The dried ($MgSO_4$) organic fraction was evaporated to give methyl 4-(3,5-dimethyl-2-formyl-4-methoxyphenyl)butyrate as a pale yellow oil. Pmr spectrum ($CDCl_3$; δ in ppm): 1.69-2.98 (6H,m); 2.31 (3H,s); 2.52 (3H,s); 3.67 (3H,s); 3.69 (3H,s); 6.91 (1H,s); 10.50 (1H, s).

v) Methyl 4-(3,5-dimethyl-2-formyl-4-methoxyphenyl)butyrate (9.4 g) and 1-triphenylphosphoranylidene-2-propanone (21 g equiv) were heated and stirred at reflux in toluene for 48 hr. The solvent was evaporated under reduced pressure and the residue was purified by column chromatography over silica with dichloromethane elution to give methyl 4-[2-(but-1-en-3-one-1-yl)-3,5-dimethyl-4-methoxyphenyl]-butyrate as a pale yellow oil. Pmr spectrum ($CDCl_3$; $\delta$ in ppm): 1.83-2.69 (6H,m); 2.25 (3H,s); 2.28 (3H,s); 2.41 (3H,s); 3.66 (3H,s); 3.70 (3H,s); 6.26 (1H,d); 6.89 (1H,s); 7.65 (1H,d).

vi) Methyl 4-(2-(but-1-en-3-one-1-yl)-3,5-dimethyl-4-methoxyphenyl)butyrate (5.0 g) and sodium dimethyl malonate (6 g equiv) were stirred and heated at reflux in dry methanol (60 ml) for 6 hr. The solvent was evaporated under reduced pressure and the residue was heated at reflux with a 10% excess of an aqueous potassium hydroxide solution (60 ml) for 8 hr. The cooled mixture was extracted with diethyl ether. The aqueous fraction was heated to 60° C and was acidified by slow addition of a dilute aqueous hydrochloric acid solution. The cooled mixture was extracted with ethyl acetate. The dried ($MgSO_4$) organic fraction was evaporated to give 5-(6-[3-(carboxy)propyl]-2,4-dimethyl-3-methoxyphenyl)-3-hydroxycyclohex-2-en-1-one as a pale yellow foam. Pmr spectrum (acetone-$d_6$; $\delta$ in ppm): 1.6-3.7 (11H,m); 2.28 (3H,m); 2.43 (3H,s); 3.65 (3H,s); 5.52 (1H,s); 6.91 (1H,s); 8.6 (2H,brs).

vii) 5-(6-[3-(Carboxy)propyl]-2,4-dimethyl-3-methoxyphenyl)-3-hydroxycyclohex-2-en-1-one (4.0 g) was slowly heated and stirred in polyphosphoric acid from 80° C to 140° C (bath temperature) over 1.5 hr. The cooled mixture was poured onto ice-water and was then extracted with ethyl acetate. The dried ($MgSO_4$) organic fraction was evaporated to give 5-(6,8-dimethyl-7-methoxy-1-oxo-1,2,3,4-tetrahydronaphth-5-yl) -3-hydroxycyclohex-2-en-1-one as a pale yellow foam.

viii) A mixture of 5-(6,8-dimethyl-7-methoxy-1-oxo-1,2,3-4-tetrahydronaphth-5-yl)-3-hydroxycyclohex-2-en-1-one (1.0 g) and propionic anhydride (2 ml) was stirred and heated at reflux in toluene (80 ml) for 1 hr. The solvent was evaporated and the residue was heated under high vacuum to remove excess anhydride. The residue was then heated at reflux with dimethylaminopyridine (0.2 g) in toluene (80 ml) for 8 hr. The solvent was evaporated and the residue was purified by column chromatography over silica with dichloromethane/ethyl acetate (9:1 v/v) elution to give 5-(6,8-dimethyl-7-methoxy-1-oxo-1,2,3,4-tetrahydronaphth5-yl)-3-hydroxy-2-propionylcylohex-2-en-1-one as a pale yellow oil. Pmr spectrum ($CDCl_3$; $\delta$ in ppm):1.17 (3H,t); 1.7-3.7 (13H,m); 2.41 (3H,s); 2.52 (3H,s); 3.66 (3H,s); 18.28 (1H,s).

ix) A mixture of 5-(6,8-dimethyl-7-methoxy-1-oxo-1,2,3,4-tetrahydronaphth-5-yl)-3-hydroxy-2-propionylcyclohex-2-en-1-one (0.48 g), ethoxyamine hydrochloride (1 equiv), anhydrous sodium acetate (1 equiv) and absolute alcohol (30 ml) was stirred at room temperature for 1 hr. The mixture was poured into a very dilute aqueous hydrochloric acid solution which was then immediately extracted with diethyl ether. The dried ($MgSO_4$) organic fraction was evaporated to give 5-(6,8-dimethyl-7-methoxy-1-oxo-1,2,3, 4-tetrahydronaphth-5-yl)-2[1-(ethoxyimino) propyl]-3-hydroxycyclohex-2-en-1-one (6) as a pale yellow oil. Pmr spectrum ($CDCl_3$; $\delta$ in ppm): 1.11-1.42 (6H,2xt); 1.9-3.8 (15H,m); 2.41 (3H,s); 2.52 (3H,s); 3.66 (3H,s); 4.14 (2H, q); OH not observed.

Example 2

5-(5,7-Dimethyl-1-oxoindan-4-yl)-2-[1-(ethoxyimino)propyl] cyclohexane-1,3-dione (7) and 5-(5,7-dimethyl-1-oxoindan-6-yl)-2-[1-(ethoxyimino)propyl] cyclohexane-1,3-dione (8).

i) A mixture of 3,5- dimethyl benzyl bromide (1 g), anhydrous potassium carbonate (0.69 g) and dimethylmalonate (0.57 ml) in dimethylfomamide (2.5 ml) was stirred overnight at room temperature. The mixture was poured into dilute hydrochloric acid and extracted with methylene chloride. The organic layer was washed with water, then dried (MgSO4) and evaporated to a colourless oil (1.0 g) which was identified as dimethyl 3,5-dimethylbenzylmalonate.

ii) A mixture of dimethyl 3,5-dimethybenzlmalonate (11.4 g, 46 m mol) and bonc acid (5.6 g, 92 m mol) was heated with stirring at 180° C for 3 hours. After cooling the mixture was dissolved in diethyl ether and water and the organic layer was dried (MgSO4) and evaporated to give a brown oil (8.6 g) which was identified as methyl 3-(3,5-dimethylphenyl) propionate. Proton magnetic resonance spectum ($CDCl_3$; $\delta$ in ppm); 2.31 (6 H, S); 2.70 (4H, d of d); 3.66 (3H, s); 6.77 (3H, s).

iii) Methyl 3-(3,5-dimethylphenyl) propionate was converted into a mixture of two isomeric 5-(5,7-dimethyl-1-oxoindanyl)-2-propionylcyclohexane-1,3-diones following essentially the same procedure as described in Example 1 parts iv) to viii). Column chromatography of the crude mixture of isomers using silica gel as adsorbent and eluting with methylene chloride allowed the separation of the pure isomers. The faster moving trione was assigned the structure 5-(5,7-dimethyl-1-oxoindan-6-yl)-2-propionylcyclo hexane-1,3-dione based on its proton magnetic resonance spectum ($CDCl_3$; $\delta$ in ppm): 1.16 (3H, t); 2.48 (3H,s); 2.75 (3H,s); 2.3 - 4.0 (11H, m); 7.14 (1H, s); 18.28 (1H,s).

iv) Each of the separated isomeric 5-(5,7-dimethyl-1-oxoindanyl) -2-propionylcyclohexane- 1,3-diones from part iii) was reacted with ethoxyamine following the general procedure described in Example 1 part ix).

5-(5,7- Dimethyl-1-oxoindan-4-yl) -2- [1-(ethoxyimino) propyl] cyclohexane-1, 3-dione (7) was isolated as a pale yellow oil, proton magnetic resonance spectrum ($CDCl_3$; $\delta$ in ppm): 1.17 (3H,t); 1.34(3H,t); 2.43-(3H,s); 2.55(3H,s); 2.3-4.0 (11 H,m); 4.12 (2H,q); 6.88(1H,s); 15.0(1H,bs).

5-(5,7-Dimethyl-1-oxoindan-6-yl) -2-[1-(ethoxyimino propyl] cyclohexane-1,3-dione (8) was isolated as a pale yellow oil, proton magnetic resonance spectrum ($CDCl_3$; $\delta$ in ppm): 1.18(3H,t); 1.34 (3H,t); 2.47-(3H,s); 2.73(3H,s); 2.4-4.0 (11H,m); 4.13(2H,q); 7.08(1H,s); 15.1(1H,bs).

Example 3

A mixture of the isomeric 5-(5,7-dimethyl-1-oxoindan-4-(and-6-) yl) -2-propionylcyclohexane-1,3-diones from Example 2 part iii) was reacted with allyloxyamine following the general procedure described in Example 1 part ix).

A mixture of 2-[1-(allyloxyimino)propyl]-5-(5,7-dimethyl-1-oxoindan-4-yl)cyclohexane-1,3-dione (9) and 2-[1-(allyloxyimino)propyl] -5-(5,7-dimethyl-1-oxoindan-6-yl) cyclohexane-1,3-dione (10) was obtained as a pale yellow oil, proton magnetic resonance spectum ($CDCl_3$; $\delta$ in ppm): 1.18(3H,t); 2.44(approx 2H,s); 2.55 (approx 2H,s); 2.46(approx 1H,s); 2.74(approx 1H,s); 2.3-3.4 (10H,m); 3.5-4.0 (1H,m); 4.52(2H,d); 5.15(2H,m); 6.0(1H,m); 6.90(0.7H,s); 7.08(0.3H,s); 15.0(1H,bs).

Example 4

Sodium salt of 5-(5,7- dimethyl-1-oxoindan-4-yl) -2-[1-(ethoxyimino)propyl] cyclohexane -1,3-dione (11).

A solution of sodium hydroxide (4 mg, 01 m mol) in water (0.5 ml) was added to a solution of 5-(5,7-dimethyl-1-oxoindan-4-yl)-2-[1-(ethoxyimino)propyl] cyclohexane-1,3-dione (7) (37 mg, 0.1 m mol) in acetone (2 ml). The mixture was stirred at room temperature briefly and then evaporated to dryness under reduced pressure. Final traces of water were removed by azeotropic distillation with toluene. The sodium salt (11) (41 mg) was isolated as a brown non-crystalline powder.

Example 5

This non-limiting Example illustrates the preparation of formulations of the compounds of the invention.

a) Emulsifiable Concentrate

Compound No 10 was dissolved in toluene containing 7% v/v "Teric" N13 and 3% v/v "Kemmat" SC15B to give an emulsifiable concentrate which may be diluted with water to the required concentration to give an aqueous emulsion which may be applied by spraying.

("Teric" is a Trade Mark and "Teric" N13, is a product of ethoxylation of nonylphenol: "Kemmat" is a Trade Mark and "Kemmat" SC15B is a formulation of calcium dodecylbenzenesulfonate.)

b) Aqueous Suspension

Compound No 11 (5 parts by weight and "Dyapol" PT (1 part by weight) were added to an aqueous solution (94 parts by weight) of "Teric" N8 and the mixture was ball milled to produce a stable aqueous suspension which may be diluted with water to the required conclentraltion to give an aqueous suspension which may be diluted with water to the required concentration to give an aqueous suspension which may be applied by spraying. ("Dyapol" is a Trade mark and "Dyapol" PT is an anionic suspending agent; "Teric" N8 is a product of ethoxylation of nonylphenol.)

c) Emulsifiable Concentrate

Compound No 10 (10 parts by weight), "Teric" N13 (5 parts by weight) and "Kemmat" SC15B (5 parts by weight) were dissolved in "Solvesso" 150 (80 parts by weight) to give an emulsifiable concentrate which may be diluted with water to the required concentrationto give an aqueous emulsion which may be applied by spraying. ("Solvesso" is a Trade Mark and "Solvesso" 150 is a high boiling point aromatic petroleum fraction.)

d) Dispersible Powder

Compound No 10 (10 parts by weight), "Matexil" DA/AC (3 parts by weight), "Aerosol" OT/B (1 part by weight) and china clay 298 (86 parts by weight) were blended and then milled to give a powder composition having a particle size below 50 microns. ("Matexil" is a Trade Mark and "Matexil" DA/AC is the disodium salt of a naphthalenesulfonic acid/formaldehyde condensate; "Aerosol" is a Trade Mark and "aerosol" OT/B is a formulation of the dioctyl ester of sodium sulfosuccinic acid.)

e) High Strength Concentrate

Compound No 10 (99 parts by weight), silica aerogel (0.5 parts by weight) and synthetic amorphous silica (0.5 parts by weight) were blended and ground in a hammer-mill to produce a powder having a particle size less than 200 microns.

f) Dusting Powder

Compound No 10 (10 parts by weight), attapulgite (10 parts by weight) and pyrophyllite (80 parts by weight) were thoroughly blended and then ground in a hammer-mill to produce a powder of particle size less than 200 microns.

Emulsifiable concentrates and/or suspensions of the compounds of the invention were prepared essentially as described in part a), b) or c) above and then diluted with water, optionally containing surface active agent and/or oil, to give aqueous compositions of the required concentration which were used, as described in Examples 6 to 8, in the evaluation of the pre-emergence and post-emergence herbicidal activity of the compounds.

Example 6

The pre-emergent herbicidal activity of the compounds of the invention formulated as described in Example 5 was assessed by the following procedure:

The seeds of the test species were sown in rows 2 cm deep in soil contained in seed boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glass house, lightly watered with an overhead spray to initiate germination and then sub-irrigated as required for optimum plant growth. After three weeks the boxes were removed from the glass house and the effect of the treatment was visually assessed. The results are presented in Table 2 where the damage to plants is rated on a scale of from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represents 100% kill. A dash (-) means that no experiment was carried out.

The names of the test plants are as follows:

| | |
|---|---|
| Wh | Wheat |
| Or | Wild Oats |
| Rg | Ryegrass |
| Jm | Japanese millet |
| B | Barley |
| P | Peas |
| Ip | Ipomea |
| Ms | Mustard |
| Sf | Sunflower |

TABLE 2

Pre-emergent Herbicidal Activity

| Compound No | Application Rate (kg/ha) | TEST PLANT Wh | Ot | Rg | Jm | B | P | Ip | Ms | Sf |
|---|---|---|---|---|---|---|---|---|---|---|
| 6 | 1.0 | 5 | 3 | 5 | 5 | 4 | 0 | 0 | 0 | 0 |
| 7 | 0.25 | 0 | 5 | 5 | 5 | 0 | 0 | 0 | 0 | 0 |
| 8 | 0.25 | 0 | 0 | 5 | 0 | 0 | 0 | 0 | 0 | 0 |

Example 7

The post-emergent herbicidal activity of the compounds of the invention formulated as described in Example 5 was assessed by the following procedure.

The seeds of the test species werd sown in rows 2 cm deep in soil contained in seed boxes. The monocotyledonous plants and the dicotyledonous plants were down in separate seed boxes in duplicate. The four seed boxes were placed in a glass house, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glass house and sprayed with the required quantity of a composition of the invention. After spraying the boxes were returned to the glass house for a further 3 weeks and the effect of treatment was visually assessed by comparison with the untreated controls. The results are presented in Table 3 where the damage to plants is rated on a scale of from 0 to 5 where 0 represents from 0 to 10% damage, 1 represents from 11 to 30% damage, 2 represents from 31 to 60% damage, 3 represents from 61 to 80% damage, 4 represents from 81 to 99% damage and 5 represnts 100% kill. A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| B | Barley |
| P | Peas |
| Ip | Ipomea |
| Ms | Mustard |
| Sf | Sunflower |

TABLE 3

Post-emergent Herbicidal Activity

| Compound No | Application Rate (kg/ha) | TEST PLANT | | | | | | | | |
|---|---|---|---|---|---|---|---|---|---|---|
| | | Wh | Ot | Rg | Jm | B | P | Ip | Ms | Sf |
| 6 | 1.0 | 5 | 5 | 5 | 5 | 5 | 0 | 0 | 0 | 0 |
| 6 | 0.25 | 4 | 4 | 1 | 5 | 5 | - | - | - | - |
| 7 | 0.25 | 5 | 5 | 5 | 5 | 5 | 0 | 0 | 0 | 0 |
| 7 | 0.0625 | 5 | 5 | 5 | 5 | 5 | - | - | - | - |
| 7 | 0.03125 | 2 | 5 | 5 | 5 | 5 | - | - | - | - |
| 8 | 0.2525 | 5 | 5 | 5 | 5 | 5 | 0 | 0 | 0 | 0 |
| 8 | 0.0625 | 5 | 5 | 5 | 5 | 5 | - | - | - | - |
| 8 | 0.03125 | 4 | 4 | 5 | 5 | 5 | - | - | - | - |

Example 8

The compounds were formulated for test by mixing an appropriate amount with 5 ml of an emulsion prepared by diluting 160 ml of a solution containing 21.9 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion

containing a test compound was then diluted to 40 ml with water and sprayed on to young pot plants (post-emergence test) of the species named in Table 4 below. Damage to test plants was assessed after 14 days on a scale of 0 to 5 wherein 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal camage was carried out after 21 days using the same scale of 0 to 5 as the post-emergence test. In both cases the degree of herbicidal damage was assessed by comparision with untreated control palnts. The results are given in Table 4 below. A dash (-) means no experiment was carried out.

The names of the test plants were as follows:

| | |
|---|---|
| Mz | Maize |
| Ww | Winter wheat |
| Rc | Rice |
| Br | Barley |
| Av | Avena fatua |
| Dg | Digitaria sanguinalis |
| Al | Alopecurus myosuroides |
| St | Setaria viridis |
| Ec | Echinochloa crus-galli |
| Sh | Sorghum halepense |
| Ag | Agropyron repens |

TABLE 4

Post-emergent Herbicidal Activity

| Compound No | APPLICATION Method Rate (kg/ha) | TEST PLANT Mz | Ww | Rc | Br | Av | Dg | Al | St | Ec | Sh | Ag |
|---|---|---|---|---|---|---|---|---|---|---|---|---|
| 6 | 0.20 | 5 | 4 | 5 | 5 | 4 | 2 | 1 | 5 | 5 | 3 | 4 |
| 6 | 0.10 | 5 | 4 | 4 | 5 | 4 | 1 | 1 | 2 | 5 | 1 | 1 |
| 7 | 0.20 | 4 | 4 | 3 | 4 | 4 | 4 | 4 | 5 | 5 | 5 | 4 |
| 7 | 0.10 | 4 | 3 | 2 | 4 | 4 | 4 | 4 | 5 | 5 | 4 | 4 |
| 7 | 0.05 | 1 | 1 | 0 | 3 | 3 | 4 | 3 | 3 | 4 | 1 | 1 |
| 7 | 0.02 | 0 | 0 | 0 | 2 | 0 | 3 | 1 | 2 | 3 | 1 | 1 |
| 8 | 0 20 | 5 | 4 | 3 | 5 | 4 | 4 | 4 | 5 | 5 | 5 | 4 |
| 8 | 0.10 | 5 | 3 | 1 | 4 | 4 | 4 | 4 | 4 | 4 | 5 | 4 |
| 8 | 0.05 | 3 | 0 | 0 | 3 | 2 | 3 | 2 | 4 | 4 | 1 | 2 |

## Claims

**1.** A compound of formula I.

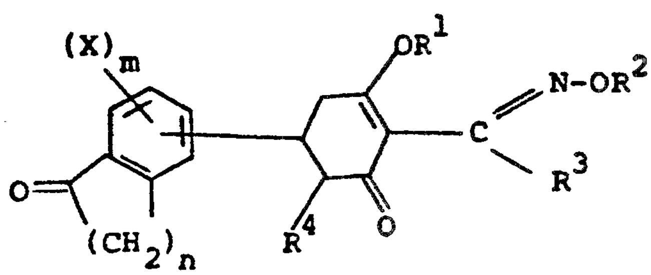

I

wherein:

m is zero or an integer selected from 1 to 3;

n is an integer selected from 2 to 4;

X, which may be the same or different, or independently selected from the group consisting of: halogen, $C_1$ to $C_6$ alkyl, $C_1$ to $C_6$ alkoxy, and $C_1$ to $C_6$ alkylthio;

$R^1$ is selected from the group consisting of: hydrogen, an acyl group, and an inorganic or organic cation;

$R^2$ is selected from the group consisting of: $C_1$ to $C_6$ alkyl; $C_2$ to $C_6$ alkenyl; $C_2$ to $C_6$ haloalkenyl; $C_2$ to $C_6$ alkynyl; $C_3$to $C_6$ haloalkynyl; and substituted $C_1$ to $C_6$ alkyl wherein the alkyl group is substituted with a substituent selected from the group consisting of halogen, $C_1$ to $C_6$ alkoxy, $C_1$ to $C_6$ alkylthio, phenyl and substituted phenyl wherein the benzene ring is substituted with from one to three substituents selected from the group consisting of halogen, $C_1$ to $C_6$ alkyl, $C_1$ to $C_6$ haloalkyl, $C_1$ to $C_6$ alkoxy, and $C_1$ to $C_6$ alkylthio;

$R^3$ is selected from the group consisting of: $C_1$ to $C_6$ alkyl; $C_1$ to $C_6$ fluoroalkyl; $C_2$ to $C_6$ alkenyl; $C_2$ to $C_6$ alkynyl; and phenyl; and

$R^4$ is selected from the group consisting of: hydrogen; halogen; cyano; $C_1$ to $C_6$ alkyl; and ($C_1$ to $C_6$ alkoxy) carbonyl.

**2.** A compound according to claim 1 wherein:

m is zero or an integer selected from 1 to 3;

n is an integer selected from 2 to 4;

X, which may be the same or different, are independently selected from the group consisting of: halogen, $C_1$ to $C_6$ alkyl, $C_1$ to $C_6$ alkoxy and $C_1$ to $C_6$ alkylthio;

$R^1$ is selected from the group consisting of: hydrogen; $C_2$ to $C_6$ alkanoyl; benzoyl and substituted benzoyl wherein the benzene ring is substituted with from one to three substituents selected from the group consisting of halogen, nitro, $C_1$ to $C_6$ alkyl and $C_1$ to $C_6$ alkoxy; benzenesulfonyl and substituted benzenesulfonyl wherein the benzene ring is substituted with from one to three substituents selected from the group consisting of halogen, nitro, $C_1$ to $C_6$ alkyl and $C_1$ $C_6$ alkoxy; and an inorganic or an organic cation selected from the alkali metals, the alkaline earth metals the transition metals the ammonium ion and the tri- and tetra(alkyl) ammonium ions wherein alkyl is selected from $C_1$ to $C_6$ alkyl and $C_1$ to $C_6$ hydroxyalkyl;

$R^2$ is selected from the group consisting of: $C_1$ to $C_6$ alkyl; $C_2$ to $C_6$ alkenyl; $C_2$ to $C_6$ haloalkenyl; $C_2$ to $C_6$ alkynyl, $C_3$ to $C_6$ haloalkynyl; and substituted $C_1$ to $C_6$ alkyl wherein the alkyl group is substituted with a substituent selected from the group consisting of halogen, $C_1$ to $C_6$ alkoxy, $C_1$ to $C_6$ alkylthio, phenyl and substituted phenyl wherein the benzene ring is substituted with from one to three substituents selected from the group consisting of halogen, $C_1$ to $C_6$ alkyl, $C_1$ to $C_6$ haloalkyl, $C_1$ to $C_6$ alkoxy, and $C_1$ to $C_6$ alkylthio;

$R^3$ is selected from the group consisting of: $C_1$ to $c_6$ alkyl; $C_1$ to $C_6$ fluoroalkyl; $C_2$ to $C_6$ alkenyl; $C_2$ to $C_6$ alkynyl; and phenyl; and

$R_4$ is selected from the group consisting of: hydrogen; halogen; cyano; $C_1$ to $C_6$ alkyl; and ($C_1$ to $C_6$ alkoxy) carbonyl.

**3.** A compound according to claim 1 or 2 wherein:

m is zero or an integer selected from 1 to 3;

n is an integer selected from 2 to 4;

X, which may be the same or different, are independently selected from the group consisting of: halogen, $C_1$ to $C_6$ alkyl, $C_1$ to $C_6$ alkoxy, and $C_1$ to $C_6$ alkylthio;

$R^1$ is selected from the group consisting of: hydrogen; $C_1$ to $C_6$ alkanoyl; benzoyl and substituted benzoyl wherein the benzene ring is substituted with from one to two substituents selected from the group consisting of halogen, nitro, $C_1$ to $C_6$ alkyl and $C_1$ to $C_6$ alkoxy; an alkali metal cation; an alkaline earth metal cation; a transition metal cation; the ammonium ion and the tri and tetra(alkyl) ammonium ions wherein alkyl is selected from $C_1$ to $C_6$ alkyl and $C_1$ to $C_6$ hydroxy-alkyl;

$R^2$ is selected from the group consisting of $C_1$ to $C_6$ alkyl, $C_1$ to $C_6$ haloalkyl, $C_2$ to $C_6$ alkenyl, $C_2$ to $C_6$ haloalkenyl and $C_3$ to $C_6$ alkynyl;

$R^3$ is selected from the group consisting of $C_1$ to $C_6$ alkyl;

$R^4$ is selected from the group consisting hydrogen, halogen, $C_1$ to $C_6$ alkyl; and ($C_1$ to $C_6$ alkoxy) carbonyl.

**4.** A compound according to any one of claims 1 to 3 inclusive wherein:

m is zero or an integer selected from 1 to 3;

n is an integer selected from 2 to 4;

X, which may be the same or different, are independently selected from the group consisting of: halogen, $C_1$ to $C_6$ alkyl, $C_1$ to $C_6$ alkoxy, and $C_1$ to $C_6$ alkylthio;

$R^1$ is selected from the group consisting of: hydrogen; $C_2$ to $C_6$ alkanoyl; benzoyl; an alkali metal cation; an alkaline earth metal cation; a transition metal cation; the ammonium ion and the tri and tetra (alkyl) ammonium ions wherein alkyl is selected from $C_1$ to $C_6$ alkyl;

$R^2$ is selected from the group consisting of $C_1$ to $C_6$ alkyl, $C_1$ to $C_6$ haloalkyl, $C_2$ to $C_6$ alkenyl, $C_2$ to $C_6$ haloalkenyl and $C_3$ to $C_6$ alkynyl;

$R^3$ is selected from the group consisting of $C_1$ to $C_6$ alkyl; and

$R^4$ is hydrogen

**5.** A compound according to any one of claims 1 to 4 inclusive wherein:

m is zero or an integer selected from 1 to 3;

n is an integer selected from 2 to 4;

X, which may be the same or different, are independently selected from the group consisting of: halogen, $C_1$ to $C_4$ alkyl, $C_1$ to $C_4$ alkoxy and $C_1$ to $C_4$ alkylthio;

$R^1$ is selected from the group consisting of: hydrogen; the alkali metal cations; the alkaline earth metal cations and the transition metal cations;

$R^2$ is selected from the group consisting of $C_1$ to $C_4$ alkyl, $C_1$ to $C_4$ haloalkyl, $C_2$ to $C_4$ alkenyl, $C_2$ to $C_4$ haloalkenyl and $C_3$ to $C_4$ alkynyl;

$R^3$ is selected from the group consisting of $C_1$ to $C_6$ alkyl; and

$R^4$ is hydrogen.

**6.** A compound according to any one of claims 1 to 5 inclusive wherein:

m is an integer selected from 1 to 3;

n is an integer selected from 2 to 4;

X, which may be the same or different, are independently selected from the group consisting of: $C_1$ to $C_4$ alkyl and $C_1$ to $C_4$ alkoxy;

$R^1$ is selected from the group consisting of hydrogen and the alkali metal cations;

$R^2$ is selected from the group consisting of $C_1$ to $C_4$ alkyl, $C_2$ to $C_4$ alkenyl, $C_2$ to $C_4$ haloalkenyl and $C_3$ to $C_4$ alkynyl.

$R^3$ is selected from $C_1$ to $C_4$ alkyl; and

$R^4$ is hydrogen.

**7.** A compound according to any one of claims 1 to 6 inclusive wherein:

m is an integer selected from 1 to 3;

n is an integer selected from 2 and 3;

X, which may be the same or different, are independently selected from the group consisting of methyl, ethyl, methoxy and ethoxy;

$R^1$ is selected from the group consisting of: hydrogen, and the sodium and potassium cations;

$R^2$ is selected from the group consisting of $C_1$ to $C_3$ alkyl, 2-haloethyl, allyl, 3-haloallyl and propargyl;

$R^3$ is selected from $C_1$ to $C_4$ alkyl; and

$R^4$ is hydrogen.

**8.** A compound according to any one of claims 1 to 7 inclusive wherein:

m is an integer selected from 2 and 3;

n is an integer selected from 2 and 3;

X, which may be the same or different, are independently selected from the group consisting of methyl and methoxy;

$R^1$ is selected from hydrogen and the sodium cation;

$R^2$ is selected from $C_1$ to $C_3$ alkyl, allyl and propargyl;

$R^3$ is selected from methy, ethyl and n-propyl;

$R^4$ is hydrogen.

**9.** A compound according to any one of claims 1 to 8 inclusive selected from the group consisting of:

5-(6,8-dimethyl-7-methoxy-1-oxo-1,2,3,4-tetrahydronaphth-5-yl)-2-[1-(ethoxyimino)propyl]-cyclohexan - 1,3-dione;

5-(5,7 -dimethyl-1-oxoindan-4-yl) -2-[1-(ethoxyimino) propyl] cyclohexane-1,3-dione;

5-(5,7-dimethyl-1-oxoindan-6-yl)-2[1-(ethoxyimino) propyl] cyclohexane- 1,3-dione;

5-(5,7-dimethyl-1-oxoindan-4-yl)-2-[1-(allyloxyimino) propyl] cyclohexane -1,3-dione;

5-(5,7-dimethyl-1-oxoindan-6-yl)-2-[1-(allyloxyimino) propyl] cyclohexane -1,3-dione.

**10.** A herbicidal composition comprising as active ingredient a compound as defined according to any one of claims 1 to 9 inclusive and a carrier therefor.

**11.** A process for severely damaging or killing unwanted plants which process comprises applying to said plants, or to the growth medium of said plants, an effective amount of a compound as defined according to any one of claims 1 to 9 inclusive or an effective amount of a composition as defined according to claim 10.

**12.** A process for selectively controlling the growth of monocotyledonous weeds in dicotyledonous crops which process comprises applying to said crop, or to the growth medium of said crop, a compound as defined according to any one of claims 1 to 9 inclusive or a composition as defined according to claims 1 to 9 inclusive or a composition as defined according to claim 10 in a amount sufficient to severely damage or kill said weeds but insufficient to substantially damage said crop.

**13.** A process according to claim 11 to claim 12 wherein the compound is applied at a rate in the range of from 0.005 to 20 kilograms per hectare.

**14.** A process for the synthesis of a compound of formula I as defined according to any one of claims 1 to 9 inclusive which process comprises reacting a 2-acyl -5-aryl cyclohexane-1,3-dione derivative of formula XIV with an alkoxyamine derivative of formula XVIII to give a compound of the invention of formula II or reacting the 2-acyl-5-aryl cyclohexane-1,3-dione derivative of formula XIV with hydroxylamine and alkylating the oxime intermediate of formula XIX with an alkylating agent of formula XX, wherein L is a leaving group, to give a compound of the invention of formula II; and optionally

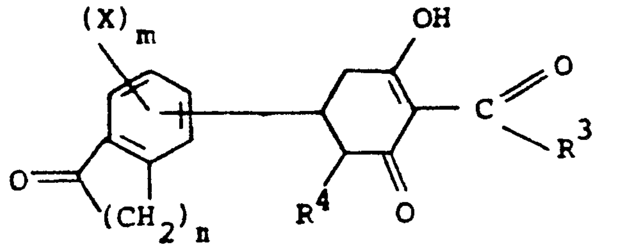

XIV

$H_2NOR^2$

XVIII

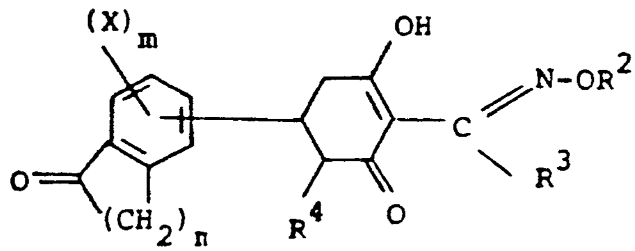

II

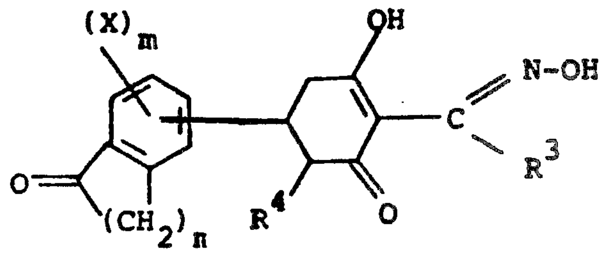

XIX

$R^2L$ $R^1L$

XX XXI

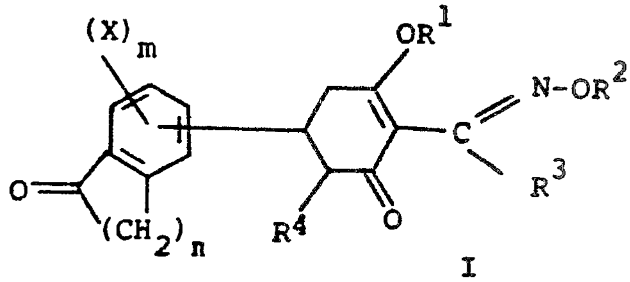

I

reacting the compound of the invention of formula II with a compound of formula XXI wherein L is a leaving group, to give a compound of the invention of formula I.

**15.** A process for the synthesis of a compound of formula X wherein

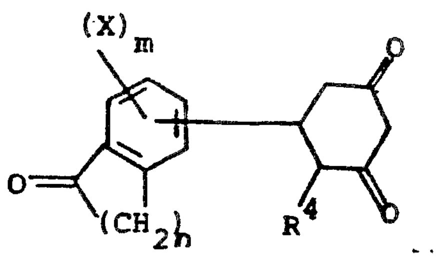

X

m and n are integers selected independently from 2 and 3; x is methyl or methoxy; which process comprises reacting a 5-(carboxy-alkylphenyl) cyclohexane-1,3-dione of formula IX wherein $R^5$ is a hydrocarbyl group, preferably a $C_{1-6}$ alkyl group, with an acidic dehydrating agent to give an intermediate compound of the invention of formula X.

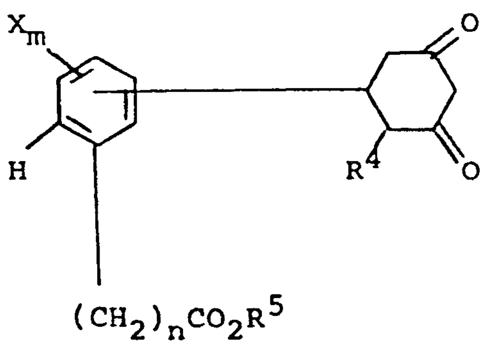

IX

## Revendications

**1.** Composé de formule I

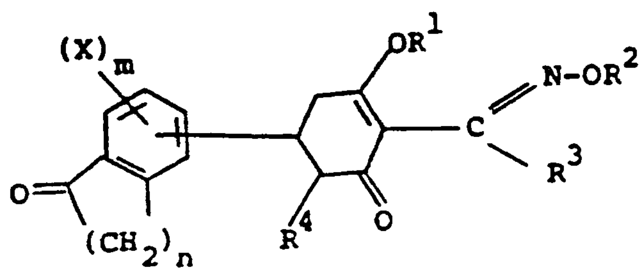

I

dans laquelle :
m est égal à zéro ou à un nombre entier ayant une valeur de 1 à 3 ;
n est un nombre entier ayant une valeur de 2 à 4 ;
les substituants X, qui peuvent être identiques ou différents, sont choisis, indépendamment, dans le groupe des substituants halogéno, alkyle en $C_1$ à $C_6$, alkoxy en $C_1$ à $C_6$ et alkylthio en $C_1$ à $C_6$ ;
$R^1$ est choisi dans le groupe constitué de l'hydrogène, d'un groupe acyle et d'un cation inorganique ou organique ;
$R^2$ est choisi dans le groupe comprenant des radicaux alkyle en $C_1$ à $C_6$, alcényle en $C_2$ à $C_6$, halogénalcényle en $C_2$ à $C_6$, alcynyle en $C_2$ à $C_6$, halogénalcynyle en $C_3$ à $C_6$ et alkyle en $C_1$ à $C_6$ substitué dont le groupe alkyle porte un substituant choisi entre des substituants halogéno, alkoxy en $C_1$ à $C_6$, alkylthio en $C_1$ à $C_6$, phényle et phényle substitué dont le noyau benzénique porte un à trois substituants choisis dans le groupe des substituants halogéno, alkyle en $C_1$ à $C_6$, halogénalkyle en $C_1$ à $C_6$, alkoxy en $C_1$ à $C_6$ et alkylthio en $C_1$ à $C_6$ ;
$R^3$ est choisi dans le groupe comprenant des radicaux alkyle en $C_1$ à $C_6$, fluoralkyle en $C_1$ à $C_6$, alcényle en $C_2$ à $C_6$, alcynyle en $C_2$ à $C_6$ et phényle ; et
$R^4$ est choisi dans le groupe comprenant l'hydrogène, un halogène, un radical cyano, alkyle en $C_1$ à $C_6$ et (alkoxy en $C_1$ à $C_6$)carbonyle.

**2.** Composé suivant la revendication 1, dans lequel :
m est égal à zéro ou a un nombre entier ayant une valeur de 1 à 3 ;
n est un nombre entier ayant une valeur de 2 à 4 ;
les substituants X, qui peuvent être identiques ou différents, sont choisis, indépendamment, dans le groupe des substituants halogéno, alkyle en $C_1$ à $C_6$, alkoxy en $C_1$ à $C_6$ et alkylthio en $C_1$ à $C_6$ ;
$R^1$ est choisi dans le groupe comprenant l'hydrogène, un radical alcanoyle en $C_2$ à $C_6$, benzoyle et

benzoyle substitué dont le noyau benzénique porte un à trois substituants choisis dans le groupe des substituants halogéno, nitro, alkyle en $C_1$ à $C_6$ et alkoxy en $C_1$ à $C_6$ ; benzènesulfonyle et benzènesulfonyle substitué dont le noyau benzénique porte un à trois substituants choisis dans le groupe des substituants halogéno, nitro, alkyle en $C_1$ à $C_6$ et alkoxy en $C_1$ à $C_6$ ; et un cation inorganique ou un cation organique choisi entre les métaux alcalins, les métaux alcalino-terreux, les métaux de transition, l'ion ammonium et les ions tri- et tétra-(alkyl)-ammonium dont les radicaux alkyle sont choisis dans le groupe des radicaux alkyle en $C_1$ à $C_6$ et hydroxyalkyle en $C_1$ à $C_6$ ;
$R^2$ est choisi dans le groupe comprenant des radicaux alkyle en $C_1$ à $C_6$, alcényle en $C_2$ à $C_6$, halogénalcényle en $C_2$ à $C_6$, alcynyle en $C_2$ à $C_6$, halogénalcynyle en $C_3$ à $C_6$ et alkyle en $C_1$ à $C_6$ substitué dont le groupe alkyle porte un substituant choisi dans le groupe des substituants halogéno, alkoxy en $C_1$ à $C_6$, alkylthio en $C_1$ à $C_6$, phényle et phényle substitué dont le noyau benzénique porte un à trois substituants choisis dans le groupe des substituants halogéno, alkyle en $C_1$ à $C_6$, halogénalkyle en $C_1$ à $C_6$, alkoxy en $C_1$ à $C_6$ et alkylthio en $C_1$ à $C_6$ ;
$R^3$ est choisi dans le groupe comprenant des radicaux alkyle en $C_1$ à $C_6$, fluoralkyle en $C_1$ à $C_6$, alcényle en $C_2$ à $C_6$, alcynyle en $C_2$ à $C_6$, et phényle ; et
$R^4$ est choisi dans le groupe comprenant l'hydrogène, un halogène, un radical cyano, alkyle en $C_1$ à $C_6$ et (alkoxy en $C_1$ à $C_6$)carbonyle.

**3.** Composé suivant la revendication 1 ou 2, dans lequel :
m est égal à zéro ou à un nombre entier ayant une valeur de 1 à 3 ;
n est un nombre entier ayant une valeur de 2 à 4 ;
les substituants X, qui peuvent être identiques ou différents, sont choisis, indépendamment, dans le groupe des substituants halogéno, alkyle en $C_1$ à $C_6$, alkoxy en $C_1$ à $C_6$ et alkylthio en $C_1$ à $C_6$ ;
$R^1$ est choisi dans le groupe comprenant l'hydrogène, un radical alcanoyle en $C_2$ à $C_6$, benzoyle et benzoyle substitué dont le noyau benzénique porte un ou deux substituants choisis dans le groupe des substituants halogéno, nitro, alkyle en $C_1$ à $C_6$ et alkoxy en $C_1$ à $C_6$ ; un cation de métal alcalin ; un cation de métal alcalinoterreux ; un cation de métal de transition ; l'ion ammonium et les ions tri- et tétra-(alkyl)-ammonium dont le radical alkyle est choisi entre des radicaux alkyle en $C_1$ à $C_6$ et hydroxyalkyle en $C_1$ à $C_6$ ;
$R^2$ est choisi dans le groupe comprenant des radicaux alkyle en $C_1$ à $C_6$, halogénalkyle en $C_1$ à $C_6$, alcényle en $C_2$ à $C_6$, halogénalcényle en $C_2$ à $C_6$ et alcynyle en $C_2$ à $C_6$ ;
$R^3$ est choisi dans le groupe des radicaux alkyle en $C_1$ à $C_6$ ;
$R^4$ est choisi dans le groupe comprenant l'hydrogène, un halogène, un radical alkyle en $C_1$ à $C_6$ et un radical (alkoxy en $C_1$ à $C_6$)carbonyle.

**4.** Composé suivant l'une quelconque des revendications 1 à 3 inclus, dans lequel :
m est égal à zéro ou à un nombre entier ayant une valeur de 1 à 3 ;
n est un nombre entier ayant une valeur de 2 à 4 ;
les substituants X, qui peuvent être identiques ou différents, sont choisis, indépendamment, dans le groupe des substituants halogéno, alkyle en $C_1$ à $C_6$, alkoxy en $C_1$ à $C_6$ et alkylthio en $C_1$ à $C_6$ ;
$R^1$ est choisi dans le groupe comprenant l'hydrogène ; un radical alcanoyle en $C_2$ à $C_6$, benzoyle, un cation de métal alcalin, un cation de métal alcalinoterreux, un cation de métal de transition, l'ion ammonium et les ions tri- et tétra-(alkyl)-ammonium dont le radical alkyle est choisi entre des radicaux alkyle en $C_1$ à $C_6$ ;
$R^2$ est choisi dans le groupe comprenant des radicaux alkyle en $C_1$ à $C_6$, halogénalkyle en $C_1$ à $C_6$, alcényle en $C_2$ à $C_6$, halogénalcényle en $C_2$ à $C_6$ et alcynyle en $C_3$ à $C_6$ ;
$R^3$ est choisi dans le groupe des radicaux alkyle en $C_1$ à $C_6$ ; et
$R^4$ est l'hydrogène.

**5.** Composé suivant l'une quelconque des revendications 1 à 4 inclus, dans lequel :
m est égal à zéro ou à un nombre entier ayant une valeur de 1 à 3 ;
n est un nombre entier ayant une valeur de 2 à 4 ;
les substituants X, qui peuvent être identiques ou différents, sont choisis, indépendamment, dans le groupe comprenant des substituants halogéno, alkyle en $C_1$ à $C_4$, alkoxy en $C_1$ à $C_4$ et alkylthio en $C_1$ à $C_4$ ;
$R^1$ est choisi dans le groupe comprenant l'hydrogène ;les cations de métaux alcalins, les cations de métaux alcalino-terreux et les cations de métaux de transition ;
$R^2$ est choisi dans le groupe comprenant des radicaux alkyle en $C_1$ à $C_4$, halogénalkyle en $C_1$ à $C_4$,

alcényle en $C_2$ à $C_4$, halogénalcényle en $C_2$ à $C_4$ et alcynyle en $C_3$ ou $C_4$ ;
$R^3$ est choisi dans le groupe des radicaux alkyle en $C_1$ à $C_6$ ; et
$R^4$ est l'hydrogène.

6. Composé suivant l'une quelconque des revendications 1 à 5 inclus, dans lequel :
m est un nombre entier ayant une valeur de 1 à 3 ;
n est un nombre entier ayant une valeur de 2 à 4 ;
les substituants X, qui peuvent être identiques ou différents, sont choisis, indépendamment, dans le groupe des substituants alkyle en $C_1$ à $C_4$ et alkoxy en $C_1$ à $C_4$ ;
$R^1$ est choisi dans le groupe comprenant l'hydrogène et les cations de métaux alcalins ;
$R^2$ est choisi dans le groupe comprenant des radicaux alkyle en $C_1$ à $C_4$, alcényle en $C_2$ à $C_4$, halogénalcényle en $C_2$ à $C_4$ et alcynyle en $C_3$ ou $C_4$ ;
$R^3$ est choisi entre des radicaux alkyle en $C_1$ à $C_4$ ; et
$R^4$ est l'hydrogène.

7. Composé suivant l'une quelconque des revendications 1 à 6 inclus, dans lequel :
m est un nombre entier ayant une valeur de 1 à 3 ;
n est un nombre entier ayant la valeur 2 ou 3 ;
les substituants X, qui peuvent être identiques ou différents, sont choisis, indépendamment, dans le groupe des substituants méthyle, éthyle, méthoxy et éthoxy ;
$R^1$ est choisi dans le groupe comprenant l'hydrogène et les cations sodium et potassium ;
$R^2$ est choisi dans le groupe des radicaux alkyle en $C_1$ à $C_3$, 2-halogénéthyle, allyle, 3-halogénallyle et propargyle ;
$R^3$ est choisi parmi les radicaux alkyle en $C_1$ à $C_4$ ; et
$R^4$ est l'hydrogène.

8. Composé suivant l'une quelconque des revendications 1 à 7 inclus, dans lequel :
m est un nombre entier ayant la valeur 2 ou 3 ;
n est un nombre entier ayant la valeur 2 ou 3 ;
les substituants X, qui peuvent être identiques ou différents, sont choisis, indépendamment, dans le groupe des substituants méthyle et méthoxy ;
$R^1$ est choisi entre l'hydrogène et le cation sodium ;
$R^2$ est choisi entre des radicaux alkyle en $C_1$ à $C_3$, allyle et propargyle ;
$R^3$ est choisi entre les radicaux méthyle, éthyle et n-propyle ;
$R^4$ est l'hydrogène.

9. Composé suivant l'une quelconque des revendications 1 à 8 inclus, choisi dans le groupe comprenant :
la 5-(6,8-diméthyl-7-méthoxy-1-oxo-1,2,3,4-tétrahydronapht-5-yl)-2-[1-(éthoxyimino)propyl]-cyclohexane-1,3-dione ;
la 5-(5,7-diméthyl-1-oxo-indane-4-yl)-2-[1-(éthoxyimino)propyl]-cyclohexane-1,3-dione ;
la 5-(5,7-diméthyl-1-oxo-indane-6-yl)-2-[1-(éthoxyimino)propyl]-cyclohexane-1,3-dione ;
la 5-(5,7-diméthyl-1-oxo-indane-4-yl)-2-[1-(allyloxyimino)propyl]-cyclohexane-1,3-dione ;
la 5-(5,7-diméthyl-1-oxo-indane-6-yl)-2-[1-(allyloxyimino)propyl]-cyclohexane-1,3-dione.

10. Composition herbicide, comprenant comme ingrédient actif un composé tel que défini conformément à l'une quelconque des revendications 1 à 9 inclus et un support approprié.

11. Procédé pour endommager gravement ou pour détruire des plantes non désirées, procédé qui consiste à appliquer auxdites plantes ou à leur milieu de croissance une quantité efficace d'un composé tel que défini suivant l'une quelconque des revendications 1 à 9 inclus, ou une quantité efficace d'une composition telle que définie conformément à la revendication 10.

12. Procédé pour combattre sélectivement la croissance de mauvaises herbes monocotylédones dans des cultures de plantes dicotylédones, procédé qui consiste à appliquer à ladite culture ou au milieu où elles se développent, un composé tel que défini conformément à l'une quelconque des revendications 1 à 9 inclus ou une composition telle que définie conformément aux revendications 1 à 9 inclus ou une composition telle que définie conformément à la revendication 10 en une quantité suffisante pour endommager gravement ou pour détruire lesdites mauvaises herbes, mais insuffisante pour endommager gravement ladite culture.

**13.** Procédé suivant la revendication 11 ou la revendication 12, dans lequel le composé est appliqué à un taux compris dans la plage de 0,005 à 20 kilogrammes par hectare.

**14.** Procédé de synthèse d'un composé de formule I tel que défini conformément à l'une quelconque des revendications 1 à 9 inclus, procédé qui consiste à faire réagir le dérivé 2-acyl-5-arylique de cyclohexane-1,3-dione de formule XIV avec un dérivé alkoxyaminé de formule XVIII pour former un composé de l'invention de formule II, ou à faire réagir le dérivé 2-acyl-5-arylique de cyclohexane-1,3-dione de formule XIV avec l'hydroxylamine, et à alkyler l'oxime intermédiaire de formule XIX avec un agent alkylant de formule XX, dans laquelle L est un groupe partant, pour former un composé de l'invention de formule II ; et à titre facultatif, à faire réagir le composé de l'invention de formule II avec un composé de formule XXI, dans laquelle L est un groupe partant, pour former un composé de l'invention de formule I.

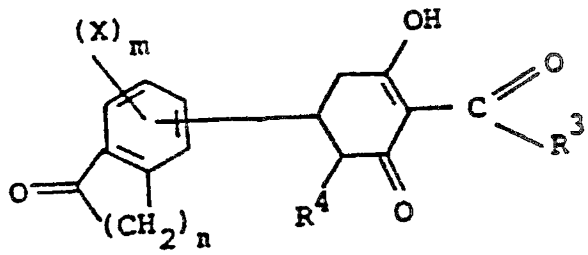

XIV

$H_2NOR^2$

XVIII

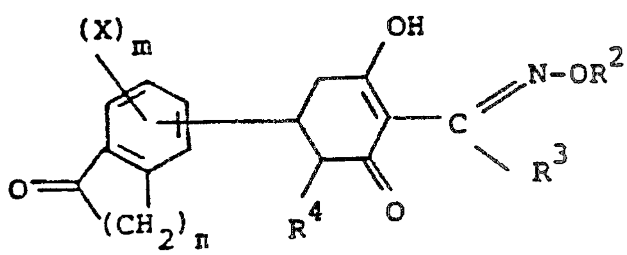

II

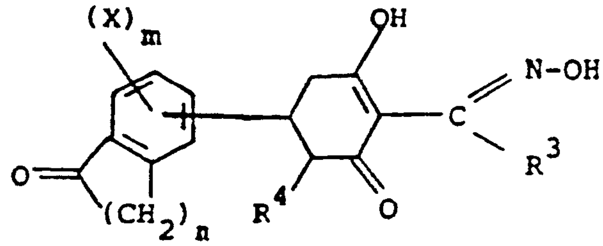

XIX

$R^2L$ $R^1L$

XX XXI

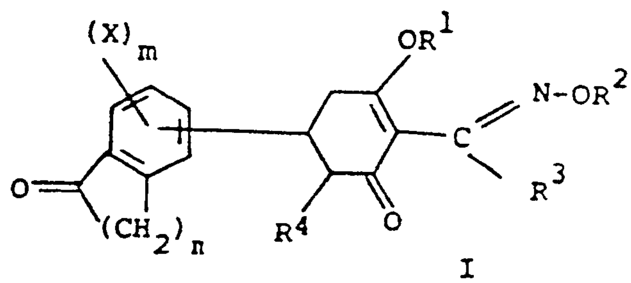

I

**15.** Procédé de synthèse d'un composé de formule X

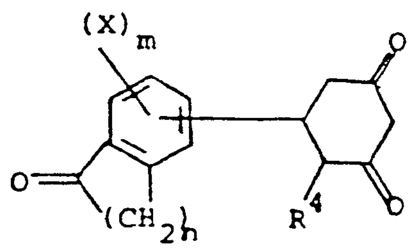

X

dans laquelle m et n sont des nombres entiers choisis, indépendamment, entre les nombres 2 et 3 ; X est un groupe méthyle ou méthoxy ; procédé qui consiste à faire réagir une 5-(carboxyalkylphényl)-cyclohexane-1,3-dione de formule IX

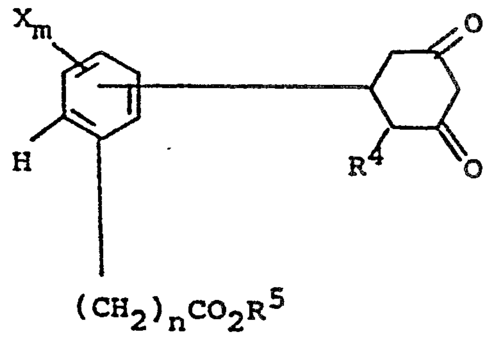

IX

dans laquelle $R^5$ est un groupe hydrocarbyle, de préférence un groupe alkyle en $C_1$ à $C_6$, avec un agent acide déshydratant pour former un composé intermédiaire de l'invention de formule X.

## Ansprüche

**1.** Verbindung der Formel I

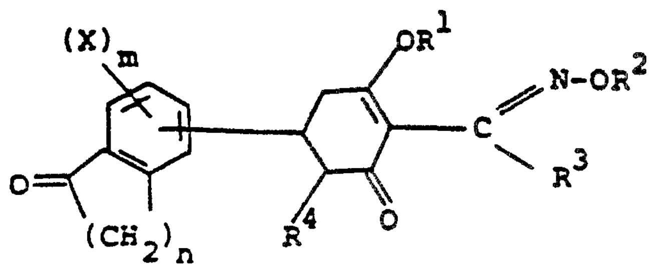

I

worin
m für Null oder eine der Ganzzahlen 1 bis 3 steht;
n für eine der Ganzzahlen 2 bis 4 steht;
X, welche gleich oder verschieden sein können, unabhängig ausgewählt sind aus Halogen, $C_{1-6}$-Alkyl, $C_{1-6}$-Alkoxy und $C_{1-6}$-Alkylthio;
$R^1$ ausgewählt ist aus Wasserstoff, den Acyl-Gruppen und den anorganischen und organischen Kationen;
$R^2$ ausgewählt ist aus $C_{1-6}$-Alkyl; $C_{2-6}$-Alkenyl; $C_{2-6}$-Halogenoalkenyl; $C_{2-6}$-Alkinyl; $C_{3-6}$-Halogenoalkinyl; und substituiertem $C_{1-6}$-Alkyl, wobei die Alkyl-Gruppe mit einem Substituenten substituiert ist, der ausgewählt ist aus Halogen, $C_{1-6}$-Alkoxy, $C_{1-6}$-Alkylthio, Phenyl und substituiertem Phenyl, wobei der Benzol-Ring mit 1 bis 3 Substituenten substituiert ist, die ausgewählt sind aus Halogen, $C_{1-6}$-Alkyl, $C_{1-6}$-Halogenoalkyl, $C_{1-6}$-Alkoxy und $C_{1-6}$-Alkylthio;
$R^3$ ausgewählt ist aus $C_{1-6}$-Alkyl, $C_{1-6}$-Fluoroalkyl, $C_{2-6}$-Alkenyl, $C_{2-6}$-Alkinyl und Phenyl;
und $R^4$ ausgewählt ist aus Wasserstoff, Halogen, Cyano, $C_{1-6}$-Alkyl und ($C_{1-6}$-Alkoxy)carbonyl.

**2.** Verbindung nach Anspruch 1, worin
m für Null oder eine der Ganzzahlen 1 bis 3 steht;
n für eine der Ganzzahlen 2 bis 4 steht;
x, welche gleich oder verschieden sein können, unabhängig ausgewählt sind aus Halogen, $C_{1-6}$-Alkyl, $C_{1-6}$-Alkoxy und $C_{1-6}$-Alkylthio;
$R^1$ausgewählt ist aus Wasserstoff; $C_{2-6}$-Alkanoyl; Benzoyl und substituiertem Benzoyl, wobei der Benzol-Ring mit 1 bis 3 Substituenten substituiert ist, die ausgewählt sind aus Halogen, Nitro, $C_{1-6}$-Alkyl und $C_{1-6}$-Alkoxy; Benzolsulfonyl und substituiertem Benzolsulfonyl, wobei der Benzol-Ring mit 1 bis 3 Substituenten substituiert ist, die ausgewählt sind aus Halogen, Nitro, $C_{1-6}$-Alkyl und $C_{1-6}$-Alkoxy; und den anorganischen und organischen Kationen, die ausgewählt sind aus den Alkalimetallen, den Erdalkalimetallen, den Übergangsmetallen, dem Ammonium-Ion und den Tri-und Tetra(alkyl)-ammonium-Ionen, wobei das Alykl ausgewählt ist aus $C_{1-6}$-Alkyl und $C_{1-6}$-Hydroxyalkyl;

$R^2$ ausgewählt ist aus $C_{1-6}$-Alkyl; $C_{2-6}$-Alkenyl; $C_{2-6}$-Halogenoalkenyl; $C_{2-6}$-Alkinyl; $C_{3-5}$-Halogenoalkinyl; und substituiertem $C_{1-6}$-Alkyl, wobei die Alkyl-Gruppe mit einem Substituenten substituiert ist, der ausgewählt ist aus Halogen, $C_{1-6}$-Alkoxy, $C_{1-6}$-Alkylthio, Phenyl und substituiertem Phenyl, wobei der Benzol-Ring mit 1 bis 3 Substituenten substituiert ist, die ausgewählt sind aus Halogen, $C_{1-6}$-Alkyl, $C_{1-6}$-Halogenoalkyl, $C_{1-6}$-Alkoxy und $C_{1-6}$-Alkylthio;
$R^3$ ausgewählt ist aus $C_{1-6}$-Alkyl, $C_{1-6}$-Fluoroalkyl, $C_{2-6}$-Alkenyl, $C_{2-6}$-Alkinyl und Phenyl; und
$R^4$ ausgewählt ist aus Wasserstoff, Halogen, Cyano, $C_{1-6}$-Alkyl und ($C_{1-6}$-Alkoxy)carbonyl.

**3.** Verbindung nach Anspruch 1 oder 2, worin
m für Null oder eine der Ganzzahlen 1 bis 3 steht;
n für eine der Ganzzahlen 2 bis 4 steht;
X, welche gleich oder verschieden sein können, unabhängig ausgewählt sind aus Halogen, $C_{1-5}$-Alkyl, $C_{1-6}$-Alkoxy und $C_{1-6}$-Alkylthio;
$R^1$ ausgewählt ist aus Wasserstoff; $C_{2-6}$-Alkanoyl; Benzoyl und substituiertem Benzoyl, wobei der Benzol-Ring mit 1 bis 2 Substituenten substituiert ist, die ausgewählt sind aus Halogen, Nitro, $C_{1-5}$-Alkyl und $C_{1-6}$-Alkoxy; den Alkalimetall-Kationen; den Erdalkalimetall-Kationen; den Übergangsmetall-Kationen; dem Ammonium-Ion und den Tri- und Tetra(alkyl)ammonium-Ionen, wobei das Alkyl ausgewählt ist aus $C_{1-6}$-Alkyl und $C_{1-6}$-Hydroxyalkyl;
$R^2$ ausgewählt ist aus $C_{1-6}$-Alkyl, $C_{1-6}$-Halogenoalkyl, $C_{2-6}$-Alkenyl, $C_{2-6}$-Halogenoalkenyl und $C_{3-5}$-Alkinyl;
$R^3$ ausgewählt ist aus $C_{1-6}$-Alkyl;
$R^4$ ausgewählt ist aus Wasserstoff, Halogen, $C_{1-6}$-Alkyl und ($C_{1-6}$-Alkoxy)carbonyl.

**4.** Verbindung nach einem der Ansprüche 1 bis 3, worin
m für Null oder eine der Ganzzahlen 1 bis 3 steht;
n für eine der Ganzzahlen 2 bis 4 steht;
X, welche gleich oder verschieden sein können, unabhängig ausgewählt sind aus Halogen, $C_{1-5}$-Alkyl, $C_{1-6}$-Alkoxy und $C_{1-6}$-Alkylthio;
$R^1$ ausgewählt ist aus Wasserstoff; $C_{2-6}$-Alkanoyl; Benzoyl; den Alkalimetall-Kationen; den Erdalkalimetall-Kationen; den Übergangsmetall-Kationen; dem Ammonium-Ion; und den Tri- und Tetra(alkyl)ammonium-Ionen, wobei das Alkyl ausgewählt ist aus $C_{1-6}$-Alkyl;
$R^2$ ausgewählt ist aus $C_{1-6}$-Alkyl, $C_{1-6}$-Halogenoalkyl, $C_{2-6}$-Alkenyl, $C_{2-6}$-Halogenoalkenyl und $C_{3-5}$-Alkinyl;
$R^3$ ausgewählt ist aus $C_{1-6}$-Alkyl; und
$R^4$ für Wasserstoff steht;

**5.** Verbindung nach einem der Ansprüche 1 bis 4, worin
m für Null oder eine der Ganzzahlen 1 bis 3 steht;
n für eine der Ganzzahlen 2 bis 4 steht;
X, welche gleich oder verschieden sein können, unabhängig ausgewählt sind aus Halogen, $C_{1-4}$-Alkyl, $C_{1-4}$-Alkoxy und $C_{1-4}$-Alkylthio;
$R^1$ ausgewählt ist aus Wasserstoff, den Alkalimetall-Kätionen, den Erdalkalimetall-Kationen und den Übergangsmetall-Kationen;
$R^2$ ausgewählt ist aus $C_{1-4}$-Alkyl, $C_{1-4}$-Halogenoalkyl, $C_{2-4}$-Alkenyl, $C_{2-4}$-Halogenoalkenyl und $C_{3-4}$-Alkinyl;
$R^3$ ausgewählt ist aus $C_{1-6}$-Alkyl;
$R^4$ für Wasserstoff steht.

**6.** Verbindung nach einem der Ansprüche 1 bis 5, worin
m für eine der Ganzzahlen 1 bis 3 steht;
n für eine der Ganzzahlen 2 bis 4 steht;
X, welche gleich oder verschieden sein können, unabhängig ausgewählt sind aus $C_{1-4}$-Alkyl und $C_{1-4}$-Alkoxy;
$R^1$ ausgewählt ist aus Wasserstoff und den Alkalimetall-Kationen;
$R^2$ ausgewählt ist aus $C_{1-4}$-Alkyl, $C_{2-4}$-Alkenyl, $C_{2-4}$-Halogenoalkenyl und $C_{3-4}$-Alkinyl;
$R^3$ ausgewählt ist aus $C_{1-4}$-Alkyl; und
$R^4$ für Wasserstoff steht.

7. Verbindung nach einem der Ansprüche 1 bis 6, worin
m für eine Ganzzahlen 1 bis 3 steht;
n für eine der Ganzzahlen 2 und 3 steht;
X, welche gleich oder verschieden sein können, unabhängig ausgewählt sind aus Methyl, Ethyl, Methoxy und Ethoxy;
$R^1$ ausgewählt ist aus Wasserstoff und den Natrium- und Kalium-Kationen;
$R^2$ ausgewählt ist aus $C_{1-3}$-Alkyl, 2-Halogenoethyl, Allyl, 3-Halogenoallyl und Propargyl;
$R^3$ ausgewählt ist aus $C_{1-4}$-Alkyl; und
$R^4$ für Wasserstoff steht.

8. Verbindung nach einem der Ansprüche 1 bis 7, worin
m für eine der Ganzzahlen 2 und 3 steht;
n für eine der Ganzzahlen 2 und 3 steht;
X, welche gleich oder verschieden sein können, unabhängig ausgewählt sind aus Methyl und Methoxy;
$R^1$ ausgewählt ist aus Wasserstoff und dem Natrium-Kation;
$R^2$ ausgewählt ist aus $C_{1-3}$-Alkyl, Allyl und Propargyl;
$R^3$ ausgewählt ist aus Methyl, Ethyl und n-Propyl; und
$R^4$ für Wasserstoff steht.

9. Verbindung nach einem der Ansprüche 1 bis 8, welche ausgewählt ist aus

5-(6,8-Dimethyl-7-methoxy-1-oxo-1,2,3,4-tetrahydronaphth-5-yl)-2-[1-(ethoxyimino)propyl]-cycloexan-1,3-dion,

5-(5,7-Dimethyl-1-oxoindan-4-yl)-2-[1-(ethoxyimino)propyl]cyclohexan-1,3-dion,

5-(5,7-Dimethyl-1-oxoindan-6-yl)-2-[1-(ethoxyimino)propyl]cyclohexan-1,3-dion,

5-(5,7-Dimethyl-1-oxoindan-4-yl)-2-[1-(allyloxyimino)propyl]cyclohexan-1,3-dion und

5-(5,7-Dimethyl-1-oxoindan-6-yl)-2-[1-(allyloxyimino)propyl]cyclohexan,1,3-dion.

10. Herbicide Zusammensetzung, welche als aktiven Bestandteil eine Verbindung nach einem der Ansprüche 1 bis 9 und einen Träger hierfür enthält.

11. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 9 oder eine wirksame Zusammensetzung nach Anspruch 10 aufgebracht wird.

12. Verfahren zur selektiven Bekämpfung des Wachstums von einkeimblättrigen Gräsern in zweikeimblättrigen Anpflanzungen, bei welchem auf die Anpflanzung oder auf das Wachstumsmedium der Anpflanzung eine Verbindung nach einem der Ansprüche 1 bis 9 oder eine Zusammensetzung nach Anspruch 10 in einer Menge aufgebracht wird, die ausreicht, die Gräser stark zu schädigen oder abzutöten, die aber nicht ausreicht, die Anpflanzung wesentlich zu schädigen.

13. Verfahren nach Anspruch 11 oder 12, bei welchem die Verbindung in einer Rate im Bereich von 0,005 bis 20 kg/ha aufgebracht wird.

14. Verfahren zur Synthese einer Verbindung der Formel 1 nach einem der Ansprüche 1 bis 9, bei welchem ein 2-Acyl-5-aryl-cyclohexan-1,3-dion-Derivat der Formel XIV mit einem Alkoxyamin-Derivat der Formel XVIII umgesetzt wird, um eine erfindungsgemäße Verbindung der Formel II herzustellen, oder das 2-Acyl-5-aryl-cyclohexan-1,3-dion-Derivat der Formel XIV mit Hydroxylamin umgesetzt wird und das Oxim-Zwischenprodukt der Formel XIX mit einem Alkylierungsmittel der Formel XX, worin L für eine abspaltbare Gruppe steht, alkyliert wird, um eine erfindungsgemäße Verbindung der Formel II herzustellen, und gegebenenfalls die erfindungsgemäße Verbindung der Formel II mit einer Verbindung der Formel XXI, worin L für eine abspaltbare Gruppe steht, umgesetzt wird, um eine erfindungsgemäße Verbindung der Formel I herzustellen.

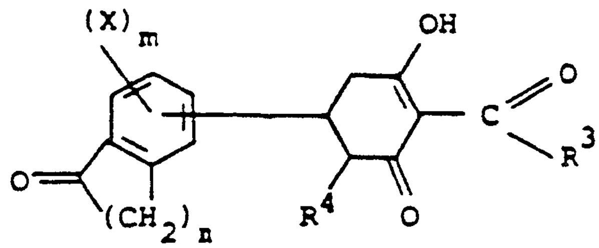

XIV

$H_2NOR^2$

XVIII

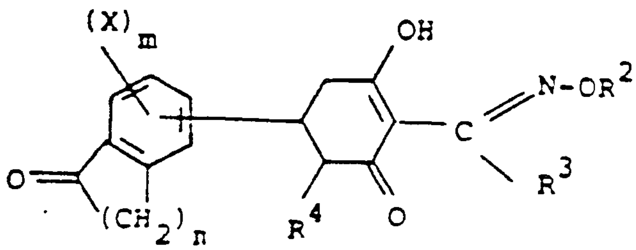

II

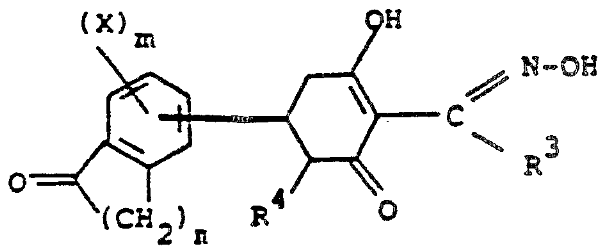

XIX

$R^2L$ $R^1L$

XX XXI

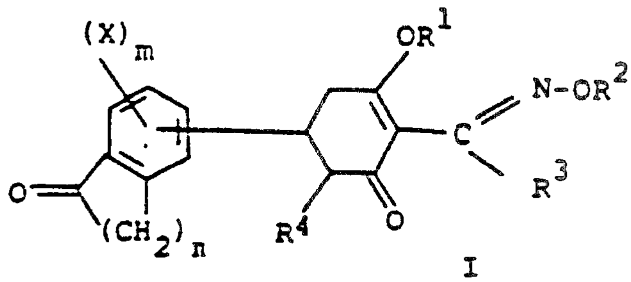

I

15. Verfahren zur Synthese einer Verbindung der Formel X

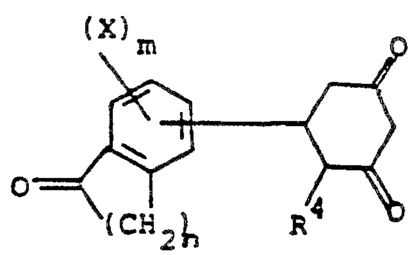

X

worin m und n unabhängig voneinander aus den Ganzzahlen 2 und 3 ausgewählt sind und X für Methyl oder Methoxy steht, bei welchem ein 5-(Carboxy-alkylphenyl)cyclohexan-1,3-dion der Formel IX,

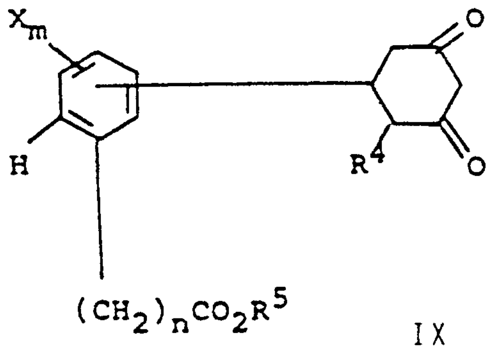

IX

worin $R^5$ für eine Kohlenwasserstoff-Gruppe, vorzugsweise eine $C_{1-6}$-Alkyl-Gruppe, steht, mit einem sauren Dehydratisierungsmittel umgesetzt wird, um eine erfindungsgemäße Zwischenproduktverbindung der Formel X herzustellen.